# EUROPEAN PATENT APPLICATION

(11) **EP 4 715 049 A1**
(43) Date of publication of application: **25.03.2026**
(21) Application number: 24807608.5
(22) Date of filing: 17.05.2024
(51) Int. Cl.: C12N 9/18, C12P 7/44, C08J 11/10

(54) **POLYPEPTIDE HAVING PET DEGRADATION ACTIVITY, CONTAINING CONSERVED RESIDUE**

(30) Priority: 18.05.2023 KR 20230064343
(71) Applicant: Zyen Co., Ltd., Daegu 41566 (KR); CJ Cheiljedang Corporation, Seoul 04560 (KR)
(72) Inventor: KIM, Kyung-Jin, Daegu 42013 (KR); JANG, Jaewon, Seoul 04560 (KR); KIM, Hyung Joon, Seoul 04560 (KR); HONG, Hwaseok, Seoul 04560 (KR); KIM, Sang Jun, Seoul 04560 (KR)
(74) Representative: Meissner Bolte Partnerschaft mbB
(86) International application number: PCT/KR2024/006752
(87) International publication number: WO 2024/237737

(57) **Abstract**

Provided are a method of identifying a polypeptide having a PET degradation activity, and a novel polypeptide having a PET degradation activity, which is identified using the corresponding method.

## Description

### [Technical Field]

The present disclosure relates to a polypeptide having a PET degradation activity.

### [Background Art]

Over 400 million tons of plastics are newly produced every year, and as environmental issues regarding waste plastics emerge, efforts are being made to reduce production thereof through regulations on single-use products and use of plastic substitutes. However, the use of plastics is actually increasing every year. PET, which accounts for less than 10% of all plastics, is newly produced at about 360 million tons annually, and is considered a plastic with the shortest life cycle because it is mainly used for single-use products. Recycling of waste plastics comprises mechanical recycling, pyrolysis, chemical recycling, etc., and each method is commercialized or entering the final research stage for commercialization. Each technology may be a solution to the waste plastic issue, but no existing method can be considered perfect due to issues such as quality deterioration by downcycling, carbon neutrality, resource depletion, the impact on eutrophication of seawater and freshwater, etc.

In order to solve environmental problems caused by waste plastics, such as microplastics, greenhouse gas emissions, and resource depletion, etc., a series of research findings are being published for degrading PET, a representative type of plastic, by using a biological technology that utilizes enzymes.

### [Prior Art Documents]

(Patent Document 1) US 10851355 B2

### [Disclosure]

### [Technical Problem]

The present disclosure relates to a method of identifying a polypeptide having a polyethylene terephthalate (PET) degradation activity, and a novel polypeptide having a PET degradation activity, which is identified using the method, a method of degrading a polyester such as PET, etc. using the polypeptide, and a method of synthesizing a polyester such as PET, etc. by recycling mono(2-hydroxymethyl) terephthalate (MHET), terephthalic acid (TPA), and/or ethylene glycol (EG) which are obtained through the degradation.

### [Technical Solution]

An object of the present disclosure is to provide a method of identifying a polypeptide having a PET degradation activity.

Another object of the present disclosure is to provide a polypeptide having a PET degradation activity.

Still another object of the present disclosure is to provide a composition comprising the polypeptide having the PET degradation activity.

Still another object of the present disclosure is to provide a polynucleotide encoding the polypeptide.

Still another object of the present disclosure is to provide a host cell comprising the polypeptide and/or the polynucleotide encoding the polypeptide; a nucleic acid construct comprising the polynucleotide; and/or a vector comprising the nucleic acid construct.

Still another object of the present disclosure is to provide a method of preparing the polypeptide having the PET degradation activity.

Still another object of the present disclosure is to provide a method of degrading a polyester, the method comprising treating the polyester with any one or more of the polypeptides having the PET degradation activity; the host cell expressing the polypeptide; and the composition comprising the polypeptide.

Still another object of the present disclosure is to provide a method of producing mono(2-hydroxymethyl) terephthalate (MHET), terephthalic acid (TPA), and/or ethylene glycol (EG), the method comprising bringing a polyester into contact with any one or more of the polypeptide having the PET degradation activity; the host cell expressing the polypeptide; and the composition comprising the polypeptide.

Still another object of the present disclosure is to provide a method of producing a polyester, the method comprising the step of synthesizing the polyester using MHET, TPA, and/or EG produced by the above method.

Still another object of the present disclosure is to provide use of the polypeptide, the host cell expressing the polypeptide, and/or the composition comprising the polypeptide in degrading PET.

Still another object of the present disclosure is to provide use of the polypeptide or the composition comprising the polypeptide for the reaction with a polyester in producing mono(2-hydroxymethyl) terephthalate (MHET), terephthalic acid (TPA), and/or ethylene glycol (EG).

### [Advantageous Effects]

By using the method of identifying a polypeptide with PET degradation activity of the present disclosure, it is possible to easily and quickly determine whether any polypeptide has PET degradation activity without performing a wet experiment. In addition, by using the polypeptide thus identified, polyesters such as PET, etc. are degraded in an environmentally friendly manner, and at the same time, degradation products with high commercial value, such as mono(2-hydroxymethyl) terephthalate (MHET), terephthalic acid (TPA), and/or ethylene glycol (EG) are produced, which may be recycled in the production of polyesters such as PET, etc.

### [Detailed Description of Preferred Embodiments]

One aspect of the present disclosure provides a method of identifying a polypeptide having a PET degradation activity.

In one specific embodiment, the method comprises the steps of preparing an amino acid sequence of a polypeptide (predicted polypeptide) predicted to have the PET degradation activity; aligning the amino acid sequence of the predicted polypeptide with the reference sequence of SEQ ID NO: 1; and determining from the alignment result whether the amino acid sequence of the predicted polypeptide has the following characteristics:
based on the reference sequence of SEQ ID NO: 1,
(1) a residue corresponding to position 62 is F, W, or Y;
(2) a residue corresponding to position 70 is P;
(3) a residue corresponding to position 81 is I, L, M, or V;
(4) a residue corresponding to position 85 is P;
(5) a residue corresponding to position 86 is G;
(6) a residue corresponding to position 94 is I, L, M, or V;
(7) a residue corresponding to position 95 is A or S;
(8) a residue corresponding to position 96 is A or S;
(9) a residue corresponding to position 98 is G;
(10) a residue corresponding to position 99 is F, W or Y;
(11) a residue corresponding to position 100 is V, L, I, or M;
(12) a residue corresponding to position 101 is V, L, I, or M;
(13) a residue corresponding to position 113 is D, N, or E;
(14) a residue corresponding to position 118 is R, Q, or K;
(15) a residue corresponding to position 135 is Q, R, E, or K;
(16) a residue corresponding to position 157 is D, N, or E;
(17) a residue corresponding to position 165 is G;
(18) a residue corresponding to position 167 is S, A, N, or T;
(19) a residue corresponding to position 169 is G;
(20) a residue corresponding to position 170 is G;
(21) a residue corresponding to position 262 is D, N, or E;
(22) a residue corresponding to position 304 is H, N, or Y;
(23) a residue corresponding to position 322 is W, F, or Y; and
(24) a residue corresponding to position 330 is D, N, or E.

In the specific embodiment, the predicted polypeptide may have a number of amino acid sequences.

Another aspect of the present disclosure provides a polypeptide having a PET degradation activity.

In one specific embodiment, the polypeptide may be a polypeptide having the PET degradation activity, and comprising an amino acid sequence, wherein
based on the reference sequence of SEQ ID NO: 1,
(1) a residue corresponding to position 62 is F, W, or Y;
(2) a residue corresponding to position 70 is P;
(3) a residue corresponding to position 81 is I, L, M, or V;
(4) a residue corresponding to position 85 is P;
(5) a residue corresponding to position 86 is G;
(6) a residue corresponding to position 94 is I, L, M, or V;
(7) a residue corresponding to position 95 is A or S;
(8) a residue corresponding to position 96 is A or S;
(9) a residue corresponding to position 98 is G;
(10) a residue corresponding to position 99 is F, W or Y;
(11) a residue corresponding to position 100 is V, L, I, or M;
(12) a residue corresponding to position 101 is V, L, I, or M;
(13) a residue corresponding to position 113 is D, N, or E;
(14) a residue corresponding to position 118 is R, Q, or K;
(15) a residue corresponding to position 135 is Q, R, E, or K;
(16) a residue corresponding to position 157 is D, N, or E;
(17) a residue corresponding to position 165 is G;
(18) a residue corresponding to position 167 is S, A, N, or T;
(19) a residue corresponding to position 169 is G;
(20) a residue corresponding to position 170 is G;
(21) a residue corresponding to position 262 is D, N, or E;
(22) a residue corresponding to position 304 is H, N, or Y;
(23) a residue corresponding to position 322 is W, F, or Y; and
(24) a residue corresponding to position 330 is D, N, or E.

In another specific embodiment, the polypeptide having the PET degradation activity comprises motifs of the following Formulae 1 to 5:

(Formula 1) X1-X2-X3-X4-X5-X6-X7-X8-P:

wherein X1 is F, W, or Y; and
X2 to X8 are arbitrary amino acids or absent;

(Formula 2) X1-X2-X3-X4-P-G:

wherein X1 is I, L, M, or V; and
X2 to X4 are arbitrary amino acids or absent;

(Formula 3) X1-X2-X3-X4-G-X5-X6-X7:

wherein X1 is I, L, M, or V;
X2 is A or S;
X3 is A or S;
X4 is an arbitrary amino acid or absent;
X5 is F, W, or Y;
X6 is V, L, I, or M; and
X7 is V, L, I, or M;

(Formula 4) X1-X2-X3-X4-X5-X6-X7-X8-G-X9-X10-X11-G-G
wherein X1 is D, N, or E;
X2 to X8, X9 and X11 are arbitrary amino acids or absent; and
X10 is S, A, N, or T;

(Formula 5) X1-X2-X3-X4-X5-X6-X7-X8-X9

wherein X1 is W, F, or Y; and
X2 to X8 are arbitrary amino acids or absent; and
X9 is D, N or E.

In any one specific embodiment of the above-mentioned specific embodiments, the polypeptide having the PET degradation activity has an amino acid sequence selected from
(i) an amino acid sequence selected from WP_109362551.1, SHM40309.1, SDZ16714.1, WP_084434874.1, WP_101366623.1, WP_117215036.1, WP_132413760.1, WP_110567505.1, WP_143590710.1, WP_091553551.1, WP_124822775.1, WP_116180173.1, OGB27210.1, WP_088988822.1, WP_125191182.1, WP_085749752.1, WP_130464304.1, WP_054022242.1, WP_082377728.1, WP_132386942.1, WP_082403176.1, WP_013736455.1, WP_073887759.1, CCH19814.1, WP_141984372.1, WP_155388305.1, WP_039906502.1, WP_137976057.1, WP_132337032.1, WP_093411427.1, WP_075737041.1, WP_139584963.1, WP_088276085.1, PIG53436.1, WP_045318561.1, WP_078759821.1, AEV21261.1, WP_047194864.1, WP_083950838.1, KWV31814.1, ADV92525.1, EOD65379.1, WP_013229249.1, WP_131056699.1, WP_072476921.1, WP_065917710.1, WP_130401777.1, WP_081971423.1, WP_086849962.1, WP_109639600.1, WP_150303381.1, WP_076469402.1, WP_086784246.1, WP_125313231.1, WP_120675340.1, WP_027346283.1, WP_155541557.1, WP_075975245.1, WP_144126986.1, WP_078844206.1, WP_091283529.1, WP_103503499.1, WP_130969140.1, WP_112669767.1, KNX38636.1, GBD22443.1, WP_083302694.1, WP_091084084.1, WP_093954453.1, WP_116813002.1, WP_089021493.1, PWK39581.1, WP_011291330.1, WP_123685830.1, WP_123748612.1, WP_084400763.1, WP_132183354.1, ADV92527.1, WP_014689902.1, WP_093935592.1, RZS34008.1, WP_138709315.1, WP_141569747.1, 1JFR_A, WP_124770494.1, 4WFI_A, WP_143737449.1, WP_080041749.1, CBY05529.1, WP_111700265.1, OKK16161.1, WP_143320421.1, WP_084578793.1, WP_104613137.1, WP_104115406.1, SPF04022.1, WP_119701604.1, WP_120326365.1, PWW60275.1, WP_086668955.1, WP_092528534.1, WP_063729606.1, 3VIS_A, WP_138698985.1, WP_145923095.1, WP_068752972.1, MBA55398.1, WP_123888242.1, WP_083724990.1, WP_044499735.1, WP_051808300.1, WP_067035690.1, WP_103564314.1, WP_129849678.1, WP_125627453.1, WP_083947829.1, WP_083303042.1, KOV66859.1, SDH02982.1, PKM30861.1, WP_103939557.1, WP_120781673.1, WP_136529585.1, WP_091310325.1, GAP50618.1, SCF11721.1, WP_130477875.1, KWV30420.1, WP_143713288.1, WP_107417268.1, WP_106966793.1, WP_092287378.1, WP_080048780.1, WP_122982115.1, PWK87198.1, KYG52038.1, OUE26840.1, WP_052685689.1, SBV25448.1, MAM88718.1, RZI82703.1, WP_107115530.1, KJK32958.1, WP_146103553.1, WP_132628273.1, ALD14029.1, RZL00883.1, WP_148029222.1, WP_105645204.1, WP_108710096.1, WP_020639922.1, and WP_132200424.1;
(ii) an amino acid sequence having 70% or more identity to the amino acid sequence of (i);
(iii) a polypeptide encoded by a polynucleotide having 70% or more sequence identity to a coding sequence of a mature polypeptide of the amino acid sequence of (i);
(iv) a polypeptide encoded by a polynucleotide that hybridizes with (a) the coding sequence of the mature polypeptide of the amino acid sequence of (i), (b) cDNA thereof, or (c) the full-length complement of (a) or (b) under low stringency conditions, medium stringency conditions, medium-high stringency conditions, high stringency conditions, or very high stringency conditions; and
(v) a functional fragment of any one polypeptide of (i) to (iv) that has the PET degradation activity; and

the polypeptide may comprise an amino acid sequence, wherein
based on the reference sequence of SEQ ID NO: 1,
   (1) a residue corresponding to position 62 is F, W, or Y;
   (2) a residue corresponding to position 70 is P;
   (3) a residue corresponding to position 81 is I, L, M, or V;
   (4) a residue corresponding to position 85 is P;
   (5) a residue corresponding to position 86 is G;
   (6) a residue corresponding to position 94 is I, L, M, or V;
   (7) a residue corresponding to position 95 is A or S;
   (8) a residue corresponding to position 96 is A or S;
   (9) a residue corresponding to position 98 is G;
   (10) a residue corresponding to position 99 is F, W or Y;
   (11) a residue corresponding to position 100 is V, L, I, or M;
   (12) a residue corresponding to position 101 is V, L, I, or M;
   (13) a residue corresponding to position 113 is D, N, or E;
   (14) a residue corresponding to position 118 is R, Q, or K;
   (15) a residue corresponding to position 135 is Q, R, E, or K;
   (16) a residue corresponding to position 157 is D, N, or E;
   (17) a residue corresponding to position 165 is G;
   (18) a residue corresponding to position 167 is S, A, N, or T;
   (19) a residue corresponding to position 169 is G;
   (20) a residue corresponding to position 170 is G;
   (21) a residue corresponding to position 262 is D, N, or E;
   (22) a residue corresponding to position 304 is H, N, or Y;
   (23) a residue corresponding to position 322 is W, F, or Y; and
   (24) a residue corresponding to position 330 is D, N, or E.

In any one specific embodiment of the above-mentioned specific embodiments, the polypeptide having the PET degradation activity has an amino acid sequence selected from (i) an amino acid sequence selected from SDZ16714.1, SHM40309.1, WP_117215036.1, WP_084434874.1, WP_116180173.1, WP_109362551.1, WP_091553551.1, WP_132413760.1, OGB27210.1, WP_101366623.1, WP_124822775.1, WP_110567505.1, WP_054022242.1, WP_130464304.1, WP_073887759.1, WP_088988822.1, WP_082377728.1, WP_132386942.1, CCH19814.1, WP_039906502.1, WP_132337032.1 and PIG53436.1, WP_082403176.1, WP_137976057.1, WP_085749752.1, WP_125191182.1, WP_141984372.1, WP_155388305.1, WP_013736455.1, WP_143590710.1, WP_139584963.1, WP_078759821.1, AEV21261.1, WP_083950838.1, KWV31814.1, ADV92525.1, and OKK16161.1; (ii) an amino acid sequence having 70% or more identity to the amino acid sequence of (i); (iii) a polypeptide encoded by a polynucleotide having 70% or more sequence identity to a coding sequence of a mature polypeptide of the amino acid sequence of (i); (iv) a polypeptide encoded by a polynucleotide that hybridizes with (a) the coding sequence of the mature polypeptide of the amino acid sequence of (i), (b) cDNA thereof, or (c) the full-length complement of (a) or (b) under low stringency conditions, medium stringency conditions, medium-high stringency conditions, high stringency conditions, or very high stringency conditions; and (v) a functional fragment of any one polypeptide of (i) to (iv) that has the PET degradation activity, and
the polypeptide may comprise an amino acid sequence, wherein
based on the reference sequence of SEQ ID NO: 1,
   (1) a residue corresponding to position 62 is F, W, or Y;
   (2) a residue corresponding to position 70 is P;
   (3) a residue corresponding to position 81 is I, L, M, or V;
   (4) a residue corresponding to position 85 is P;
   (5) a residue corresponding to position 86 is G;
   (6) a residue corresponding to position 94 is I, L, M, or V;
   (7) a residue corresponding to position 95 is A or S;
   (8) a residue corresponding to position 96 is A or S;
   (9) a residue corresponding to position 98 is G;
   (10) a residue corresponding to position 99 is F, W or Y;
   (11) a residue corresponding to position 100 is V, L, I, or M;
   (12) a residue corresponding to position 101 is V, L, I, or M;
   (13) a residue corresponding to position 113 is D, N, or E;
   (14) a residue corresponding to position 118 is R, Q, or K;
   (15) a residue corresponding to position 135 is Q, R, E, or K;
   (16) a residue corresponding to position 157 is D, N, or E;
   (17) a residue corresponding to position 165 is G;
   (18) a residue corresponding to position 167 is S, A, N, or T;
   (19) a residue corresponding to position 169 is G;
   (20) a residue corresponding to position 170 is G;
   (21) a residue corresponding to position 262 is D, N, or E;
   (22) a residue corresponding to position 304 is H, N, or Y;
   (23) a residue corresponding to position 322 is W, F, or Y; and
   (24) a residue corresponding to position 330 is D, N, or E.

In any one specific embodiment of the above-mentioned specific embodiments, the polypeptide having the PET degradation activity has an amino acid sequence selected from (i) an amino acid sequence selected from SDZ16714.1, SHM40309.1, WP_117215036.1, WP_084434874.1, WP_116180173.1, WP_109362551.1, WP_091553551.1, WP_132413760.1, OGB27210.1, WP_101366623.1, WP_124822775.1, WP_110567505.1, WP_054022242.1, WP_130464304.1, WP_073887759.1, WP_088988822.1, WP_082377728.1, WP_132386942.1, CCH19814.1, WP_039906502.1, WP_132337032.1 and PIG53436.1; (ii) an amino acid sequence having 70% or more identity to the amino acid sequence of (i); (iii) a polypeptide encoded by a polynucleotide having 70% or more sequence identity to a coding sequence of a mature polypeptide of the amino acid sequence of (i); (iv) a polypeptide encoded by a polynucleotide that hybridizes with (a) the coding sequence of the mature polypeptide of the amino acid sequence of (i), (b) cDNA thereof, or (c) the full-length complement of (a) or (b) under low stringency conditions, medium stringency conditions, medium-high stringency conditions, high stringency conditions, or very high stringency conditions; and (v) a functional fragment of any one polypeptide of (i) to (iv) that has the PET degradation activity, and
the polypeptide may comprise an amino acid sequence, wherein
based on the reference sequence of SEQ ID NO: 1,
   (1) a residue corresponding to position 62 is F, W, or Y;
   (2) a residue corresponding to position 70 is P;
   (3) a residue corresponding to position 81 is I, L, M, or V;
   (4) a residue corresponding to position 85 is P;
   (5) a residue corresponding to position 86 is G;
   (6) a residue corresponding to position 94 is I, L, M, or V;
   (7) a residue corresponding to position 95 is A or S;
   (8) a residue corresponding to position 96 is A or S;
   (9) a residue corresponding to position 98 is G;
   (10) a residue corresponding to position 99 is F, W or Y;
   (11) a residue corresponding to position 100 is V, L, I, or M;
   (12) a residue corresponding to position 101 is V, L, I, or M;
   (13) a residue corresponding to position 113 is D, N, or E;
   (14) a residue corresponding to position 118 is R, Q, or K;
   (15) a residue corresponding to position 135 is Q, R, E, or K;
   (16) a residue corresponding to position 157 is D, N, or E;
   (17) a residue corresponding to position 165 is G;
   (18) a residue corresponding to position 167 is S, A, N, or T;
   (19) a residue corresponding to position 169 is G;
   (20) a residue corresponding to position 170 is G;
   (21) a residue corresponding to position 262 is D, N, or E;
   (22) a residue corresponding to position 304 is H, N, or Y;
   (23) a residue corresponding to position 322 is W, F, or Y; and
   (24) a residue corresponding to position 330 is D, N, or E.

In any one specific embodiment of the above-mentioned specific embodiments, the polypeptide having the PET degradation activity has 80% or more sequence identity or homology to WP_039906502.1.

Still another aspect of the present disclosure provides a composition comprising the polypeptide having the PET degradation activity.

In any one specific embodiment of the above-mentioned specific embodiments, the composition may be a composition for degrading PET.

Still another aspect of the present disclosure provides a polynucleotide encoding the polypeptide having the PET degradation activity.

Still another aspect of the present disclosure provides a nucleic acid construct comprising the polynucleotide.

Still another aspect of the present disclosure provides a vector comprising the polynucleotide or the nucleic acid construct.

Still another aspect of the present disclosure provides a host cell comprising the polypeptide having the PET degradation activity, the polynucleotide, the nucleic acid construct, and/or the vector.

Still another aspect of the present disclosure provides a method of preparing the polypeptide having the PET degradation activity, the method comprising the steps of culturing the host cell; and recovering the polypeptide having the PET degradation activity which is expressed in the culturing step.

Still another aspect of the present disclosure provides a method of degrading a polyester, the method comprising bringing the polyester into contact with the polypeptide having the PET degradation activity, the host cell expressing the polypeptide having the PET degradation activity, and/or the composition comprising the polypeptide having the PET degradation activity.

In any one specific embodiment of the specific embodiments, the polyester may be PET.

Still another aspect of the present disclosure provides a method of producing mono(2-hydroxymethyl) terephthalate (MHET), terephthalic acid (TPA), and/or ethylene glycol (EG), the method comprising bringing a polyester into contact with the polypeptide having the PET degradation activity, the host cell expressing the polypeptide having the PET degradation activity, and/or the composition comprising the polypeptide having the PET degradation activity.

In any one specific embodiment of the specific embodiments, the polyester may be PET.

In any one specific embodiment of the specific embodiments, the step of recovering MHET, TPA, and/or EG produced by the above method may be further comprised.

Still another aspect of the present disclosure provides a method of synthesizing a polyester using MHET, TPA, and/or EG produced by the above method.

In any one specific embodiment of the specific embodiments, the polyester may be PET.

Still another aspect of the present disclosure provides use of the polypeptide, the host cell expressing the polypeptide, and/or the composition comprising the polypeptide in degrading PET.

Still another aspect of the present disclosure provides use of the polypeptide having the PET degradation activity and/or the composition comprising the polypeptide for the reaction with a polyester in producing mono(2-hydroxymethyl) terephthalate (MHET), terephthalic acid (TPA), and/or ethylene glycol (EG).

### [Mode for Carrying Out the Invention]

The present disclosure will be described in detail as follows. Meanwhile, each description and embodiment disclosed in the present disclosure may also be applied to other descriptions and embodiments. That is, all combinations of various elements disclosed in the present disclosure fall within the scope of the present disclosure. Further, the scope of the present disclosure is not limited by the specific description described below.

Further, those skilled in the art will recognize, or be able to ascertain using no more than routine experimentation, many equivalents to the specific embodiments of the disclosure described herein. Further, these equivalents should be interpreted to fall within the present disclosure.

Further, a number of papers and patent documents are referenced and cited throughout this specification. The disclosures of the cited papers and patent documents are incorporated herein by reference in their entirety to further clarify the level and scope of the subject matter to which the present disclosure pertains.

As used in the specification and appended claims of the present disclosure, the singular forms ("a", "an", and "the") comprise plural referents unless the context clearly states otherwise. Unless the context indicates otherwise, singular terms shall comprise the plural, and plural terms shall comprise the singular. As used in the specification and appended claims of the present disclosure, unless stated otherwise, the use of "or" may be used to comprise "and/or".

As used herein, the term "about" may be presented before a particular numerical value. The term "about" used herein comprises not only the exact number recited after the term, but also a range that is near or close to that number. Considering the context in which the number is presented, it may be determined whether any number is close to or near the particular number presented. In one example, the term "about" may refer to a range from -10% to +10% of a numerical value. In another example, the term "about" may refer to a range from -5% to +5% of a given numerical value, but is not limited thereto.

As used herein, the descriptions such as the terms "first, second, third, ...," "i), ii), iii)..." or "(a), (b), (c), (d)..." are used to distinguish similar elements, and these terms do not mean that the elements are performed continually or sequentially. For example, when the terms are used in reference to steps of a method, use, or assay, there may be no time interval between these steps, or they may be performed concurrently, or may be performed several seconds, several minutes, several hours, several days, or several months apart.

As used herein, the term "consisting essentially of" may mean that, in the case where the features of the object claimed herein are not substantially affected by the presence of an unspecified component, the unspecified component may be present.

As used herein, the term "consisting of" means that the total ratio of specific component(s) is 100%. The components or features that are recited after the term "consisting of" may be essential or mandatory. In some embodiments, in addition to the components or features recited after the term "consisting of", any other components or non-essential components may be excluded.

As used herein, the term "comprising" means the presence of features, steps or components recited after the term, and does not exclude the presence or addition of one or more features, steps or components. The components or features recited after the term "comprising" herein may be essential or mandatory. However, in some embodiments, the term may further comprise any other or non-essential components or features.

As used herein, the term "comprising" may be modified to refer to "consisting essentially of" or "consisting of" in some embodiments.

With respect to amino acid sequences in the present disclosure, although it is described as a polypeptide "comprising" an amino acid sequence described by a specific sequence, a polypeptide "consisting of" an amino acid sequence described by a specific sequence, or a polypeptide or protein "having" an amino acid sequence described by a specific sequence, it is apparent that any protein having an amino acid sequence in which part of the sequence is deleted, modified, substituted, conservatively substituted or added may be used in the present disclosure if it has the identical or corresponding activity to that of the polypeptide consisting of the amino acid sequence of the corresponding sequence. For example, it may be a case where the N-terminus and/or C-terminus of the amino acid sequence is added with a sequence that does not alter the function of the protein, a naturally occurring mutation, a silent mutation thereof, or a conservative substitution, but is not limited thereto. Meanwhile, the sequence information of the amino acid sequence described by name in the present disclosure may be found in known databases, e.g., NCBI (https://www.ncbi.nlm.nih.gov/), Uniprot, etc.

As used herein, the term "protein" or "polypeptide" refers to a polymer or oligomer of consecutive amino acid residues. In the present disclosure, "polypeptide," "protein," and "peptide" may be used interchangeably with "amino acid sequence."

In some cases, an amino acid sequence exhibiting activity may be referred to as an "enzyme". In the present disclosure, unless indicated otherwise, amino acid sequences are described in an N-terminal to C-terminal direction.

As used herein, the term "recombinant" with respect to a cell, or nucleic acid, polypeptide, or vector indicates that the cell, nucleic acid, polypeptide, or vector has been modified by the introduction of a heterologous nucleic acid or polypeptide or the alteration of a native nucleic acid or polypeptide, or that the cell is derived from a cell thus modified. Therefore, for example, recombinant cells may express genes that are not found within the native (non-recombinant) form of the cells, or may express native genes that are otherwise abnormally expressed, under expressed or not expressed at all.

As used herein, the term "isolated" refers to a substance which exists in a non-naturally occurring environment or does not exist naturally. It comprises a substance (sequence, enzyme or nucleic acid) that has been at least substantially free from a substance which is naturally associated and found in nature, for example, at least one other component having a sequence, enzyme or nucleic acid.

For example, an isolated sequence, enzyme, or nucleic acid provided herein may be provided in a form that is substantially free of one or more contaminants.

Examples of isolated substances comprise i) any substance that is non-naturally occurring, ii) any substance (e.g., enzyme, variant, nucleic acid, protein, peptide, or cofactor) from which one, or more, or all naturally-occurring components associated in nature have been removed, iii) any substance that has been artificially modified from a substance found in nature, or iv) any substance that has been modified to alter the amount of that substance relative to other components associated in nature (e.g., increase in the copy number of a gene encoding a specific substance; modification of a promoter naturally linked to a gene encoding a specific substance into a highly active promoter, etc.), but are not limited thereto.

With respect to amino acid or nucleic acid sequences in the present disclosure, the term "fragment" means a part of a parent sequence. For example, it may be a polypeptide in the form in which one or more amino acids of the parent sequence are removed from the C- or N-terminus.

As used herein, the term "fragment" of an enzyme may refer to a "functional fragment". The "functional fragment" may also be referred to as an active fragment, and means a polypeptide that is a part of a parent enzyme and has enzymatic activity of the parent enzyme. For example, the functional fragment of an enzyme may comprise a catalytic site of the enzyme.

The fragment of the enzyme may comprise a part of the full length of the parent enzyme. For example, the fragment of the enzyme may comprise amino acids of at least about 20%, about 30%, about 40%, about 50%, about 60%, about 70%, about 80%, about 90%, about 95%, about 98%, about 99% or more, or about less than 100% of the full length of the parent enzyme, but is not limited thereto.

Throughout the entire specification of the present disclosure, the conventional one-letter and three-letter codes for naturally occurring amino acids are used. Further, the amino acids mentioned as abbreviations herein are described according to the nomenclature rules of IUPAC-IUB:

| | |
|---|---|
| Alanine Ala, A | Arginine Arg, R |
| Asparagine Asn, N | Aspartic acid Asp, D |
| Cysteine Cys, C | Glutamic acid Glu, E |
| Glutamine Gln, Q | Glycine Gly, G |
| Histidine His, H | Isoleucine Ile, I |
| Leucine Leu, L | Lysine Lys, K |
| Methionine Met, M | Phenylalanine Phe, F |
| Proline Pro, P | Serine Ser, S |
| Threonine Thr, T | Tryptophan Trp, W |
| Tyrosine Tyr, Y | Valine Val, V |

Meanwhile, any amino acid may be described as Xaa or X.

Further, three-letter codes generally allowed not only for naturally occurring amino acids, but also for other amino acids, such as Aib (2-aminoisobutyric acid), Sar (N-methylglycine), α-methyl-glutamic acid, etc. may be used.

Amino acids may be generally classified based on similarity of polarity, charge, solubility, hydrophobicity, hydrophilicity, and/or amphipathic nature of a residue. Accordingly, amino acid substitution may generally occur based on similarity of polarity, charge, solubility, hydrophobicity, hydrophilicity, and/or amphipathic nature of a residue.

For example, among the amino acids having an electrically charged side chain (electrically charged amino acids), positively charged (basic) amino acids comprise arginine, lysine, and histidine, negatively charged (acidic) amino acids comprise glutamic acid and aspartic acid; among the amino acids having an uncharged side chain (uncharged amino acids), non-polar amino acids comprise glycine, alanine, valine, leucine, isoleucine, methionine, phenylalanine, tryptophan, and proline, polar or hydrophilic amino acids comprise serine, threonine, cysteine, tyrosine, asparagine, and glutamine, and among the non-polar amino acids, aromatic amino acids comprise phenylalanine, tryptophan, and tyrosine.

As used herein, the term "gene" means a polynucleotide that encodes a polypeptide and a polynucleotide comprising regions the upstream and downstream of the coding region. In some embodiments, a gene may have a sequence (intron) inserted between each coding region (exon).

As used herein, the term "homology" or "identity" refers to a degree of relevance between two given amino acid sequences or nucleotide sequences, and may be expressed as a percentage. The terms homology and identity may often be used interchangeably with each other.

The sequence homology or identity of conserved polynucleotides or polypeptides may be determined by standard alignment algorithms and may be used with a default gap penalty established by the program being used. Substantially, homologous or identical sequences may generally hybridize under moderately or highly stringent conditions to the full length of the sequence or at least about 50%, about 60%, about 70%, about 80%, or about 90% of the full-length. It is apparent that hybridization also comprises hybridization of a polynucleotide with a polynucleotide comprising a general codon or a codon in consideration of codon degeneracy.

Whether any two polynucleotide or polypeptide sequences have homology, similarity, or identity may be, for example, determined using a known computer algorithm such as the "FASTA" program using default parameters, as in Pearson et al (1988) [Proc. Natl. Acad. Sci. USA 85]: 2444. Alternatively, it may be determined by the Needleman Wunsch algorithm (Needleman and Wunsch, 1970, J. Mol. Biol. 48: 443-453), as performed using the Needleman program of the EMBOSS package (EMBOSS: The European Molecular Biology Open Software Suite, Rice et al., 2000, Trends Genet. 16: 276-277) (version 5.0.0 or later) (GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego, 1994, and [CARILLO ET al.](1988) SIAM J Applied Math 48: 1073). For example, the homology, similarity, or identity may be determined using BLAST or ClustalW of the National Center for Biotechnology Information, but is not limited thereto.

The homology, similarity, or identity of polynucleotides or polypeptides may be, for example, determined by comparing sequence information using, for example, the GAP computer program, such as Needleman et al. (1970), J Mol Biol. 48:443, as disclosed in Smith and Waterman, Adv. Appl. Math (1981) 2:482. Briefly, the GAP program defines the homology, similarity, or identity as the value obtained by dividing the number of similarly aligned symbols (i.e., nucleotides or amino acids) by the total number of the symbols in the shorter of the two sequences. Default parameters for the GAP program may comprise (1) a unitary matrix (comprising a value of 1 for identities and 0 for non-identities) and the weighted comparison matrix of Gribskov et al(1986) Nucl. Acids Res. 14: 6745, as disclosed in Schwartz and Dayhoff, eds., Atlas Of Protein Sequence And Structure, National Biomedical Research Foundation, pp. 353-358 (1979) (or EDNAFULL substitution matrix (EMBOSS version of NCBI NUC4.4)); (2) a penalty of 3.0 for each gap and an additional 0.10 penalty for each symbol in each gap (or a gap opening penalty of 10 and a gap extension penalty of 0.5); and (3) no penalty for end gaps.

Further, whether any two polynucleotide or polypeptide sequences have homology, similarity, or identity with each other may be identified by comparing the sequences in a Southern hybridization experiment under stringent conditions as defined, and appropriate hybridization conditions defined are within the skill of the art, and may be determined by a method well known to those skilled in the art (e.g., J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York), but is not limited thereto.

As used herein, the term "mature polypeptide" means a polypeptide in a form without a signal sequence or propeptide sequence. A mature protein/polypeptide/peptide may be a functional form of a protein/polypeptide/peptide. The mature polypeptide may be in a final form after translation or post-translational modification. Examples of the posttranslational modification comprise N- terminal or C-terminal modifications, glycosylation, phosphorylation, leader sequence removal etc., but are not limited thereto.

As used herein, the term "nucleic acid construct" refers to a single- or doublestranded nucleic acid molecule, which comprises one or more regulatory sequences, and which is artificially synthesized, or engineered to comprise a specific sequence in a manner that does not exist in nature, or isolated from nature.

As used herein, the term "expression" comprises any step involved in the production of a polypeptide, e.g., transcription, post-transcriptional modification, translation, post-translational modification, and secretion, etc., but is not limited thereto.

As used herein, the term "expression vector" refers to a linear or circular nucleic acid molecule comprising a coding sequence and an operably linked regulatory sequence for expression thereof.

As used herein, the term "operably linked" refers to a configuration of placing a regulatory sequence to an appropriate position to direct expression of a coding sequence. Thus, the term "operably linked" comprises an attachment or linking between a regulatory region of a functional domain having a known or desired activity such as a promoter, a terminator, a signal sequence, or an enhancer, and a target (gene or polypeptide) such that the expression, secretion, or function of the target may be regulated in accordance with the known or desired activity.

As used herein, the term "reference sequence" means a sequence used to determine the position of an amino acid within an arbitrary amino acid sequence. The arbitrary amino acid sequence may be aligned with a reference sequence to determine the position of an amino acid within the arbitrary amino acid sequence, which corresponds to a particular position of the reference sequence. In other words, the reference sequence may be used as a reference point when trying to determine the position of an amino acid in a group of arbitrary amino acid sequences that have various sequence lengths and different amino acid arrangements but comprise specific identical motifs. This is distinct from the parent sequence or backbone that is a target into which a mutation is introduced.

As used herein, the "alignment" of sequences means expressing the degree to which two or more sequences are functionally or evolutionarily related and which parts of the sequences have such relationships. Sequence alignments are created through calculations based on certain criteria, and the calculation process is complex and requires a large amount of calculations. For this reason, computer programs are used. Sequence alignments may be performed using various programs, and representatively, ClustalV, ClustalW or ClustalX may be utilized for aligning multiple sequences.

As used herein, the term "corresponding to" refers to an amino acid residue at the position recited in a protein or polypeptide, or an amino acid residue which is similar, identical, or homologous to the residue recited in a protein or polypeptide. Identifying an amino acid at a corresponding position may be determining a particular amino acid in a sequence that refers to a particular sequence. As used herein, the "corresponding region" generally refers to a similar or corresponding position in the related protein or reference protein.

In the present disclosure, the COG4188 consensus sequence of SEQ ID NO: 1 may be used as a reference sequence to determine the position of amino acids in an arbitrary amino acid sequence.

In other words, SEQ ID NO: 1 disclosed herein may be used to determine the corresponding amino acid residues in an arbitrary polypeptide having PET degradation activity. This means that the amino acid sequence of SEQ ID NO: 1 is not a parent sequence but a reference sequence. Therefore, in the present disclosure, the description such as "an amino acid corresponding to position N is G, based on SEQ ID NO: 1" is interpreted to mean only that when an amino acid sequence of an arbitrary polypeptide is aligned with the amino acid sequence of SEQ ID NO: 1, the amino acid corresponding to position N of SEQ ID NO: 1 is G in the amino acid sequence of the arbitrary polypeptide. In addition, it is not limited to the meaning that the amino acid sequence of the arbitrary polypeptide has the same residue as that of SEQ ID NO: 1 at positions other than the amino acid at position N, and is not interpreted to mean that the arbitrary polypeptide comprises the amino acid sequence of SEQ ID NO: 1.

For example, an amino acid sequence of an arbitrary polypeptide is aligned with SEQ ID NO: 1, and based on the alignment, each amino acid residue of the amino acid sequence may be numbered with reference to the numerical position of the amino acid residue corresponding to the amino acid residue of SEQ ID NO: 1. For example, a sequence alignment algorithm such as that described herein may identify the position of an amino acid or a position where modifications such as substitutions, insertions, or deletions occur, compared to a query sequence (also referred to as a "reference sequence"), or the same residues as those of the reference sequence. In an arbitrary amino acid sequence, when an amino acid at a position corresponding to a specific position of SEQ ID NO: 1 is different from that of SEQ ID NO: 1, it may be expressed that the amino acid is modified (e.g., substituted), and when the amino acid is identical, it may be expressed that the amino acid is maintained or conserved.

In the present disclosure, the position corresponding to a specific amino acid of SEQ ID NO: 1 within an arbitrary sequence is defined by displaying the results of multiple alignment of genes encoding the following 152 amino acid sequences using the ClustalW2 program (see Larkin MA, Blackshields G, Brown NP, Chenna R, McGettigan PA, McWilliam H, Valentin F, Wallace IM, Wilm A, Lopez R, Thompson JD, Gibson TJ, Higgins DG.(2007). Clustal W and Clustal X version 2.0. Bioinformatics, 23, 2947-2948, http://www.clustal.org/clustal2), based on SEQ ID NO: 1, and in this regard, the parameters are default:
WP_109362551.1, SHM40309.1, SDZ16714.1, WP_084434874.1, WP_101366623.1, WP_117215036.1, WP_132413760.1, WP_110567505.1, WP_143590710.1, WP_091553551.1, WP_124822775.1, WP_116180173.1, OGB27210.1, WP_088988822.1, WP_125191182.1, WP_085749752.1, WP_130464304.1, WP_054022242.1, WP_082377728.1, WP_132386942.1, WP_082403176.1, WP_013736455.1, WP_073887759.1, CCH19814.1, WP_141984372.1, WP_155388305.1, WP_039906502.1, WP_137976057.1, WP_132337032.1, WP_093411427.1, WP_075737041.1, WP_139584963.1, WP_088276085.1, PIG53436.1, WP_045318561.1, WP_078759821.1, AEV21261.1, WP_047194864.1, WP_083950838.1, KWV31814.1, ADV92525.1, EOD65379.1, WP_013229249.1, WP_131056699.1, WP_072476921.1, WP_065917710.1, WP_130401777.1, WP_081971423.1, WP_086849962.1, WP_109639600.1, WP_150303381.1, WP_076469402.1, WP_086784246.1, WP_125313231.1, WP_120675340.1, WP_027346283.1, WP_155541557.1, WP_075975245.1, WP_144126986.1, WP_078844206.1, WP_091283529.1, WP_103503499.1, WP_130969140.1, WP_112669767.1, KNX38636.1, GBD22443.1, WP_083302694.1, WP_091084084.1, WP_093954453.1, WP_116813002.1, WP_089021493.1, PWK39581.1, WP_011291330.1, WP_123685830.1, WP_123748612.1, WP_084400763.1, WP_132183354.1, ADV92527.1, WP_014689902.1, WP_093935592.1, RZS34008.1, WP_138709315.1, WP_141569747.1, 1JFR_A, WP_124770494.1, 4WFI_A, WP_143737449.1, WP_080041749.1, CBY05529.1, WP_111700265.1, OKK16161.1, WP_143320421.1, WP_084578793.1, WP_104613137.1, WP_104115406.1, SPF04022.1, WP_119701604.1, WP_120326365.1, PWW60275.1, WP_086668955.1, WP_092528534.1, WP_063729606.1, 3VIS_A, WP_138698985.1, WP_145923095.1, WP_068752972.1, MBA55398.1, WP_123888242.1, WP_083724990.1, WP_044499735.1, WP_051808300.1, WP_067035690.1, WP_103564314.1, WP_129849678.1, WP_125627453.1, WP_083947829.1, WP_083303042.1, KOV66859.1, SDH02982.1, PKM30861.1, WP_103939557.1, WP_120781673.1, WP_136529585.1, WP_091310325.1, GAP50618.1, SCF11721.1, WP_130477875.1, KWV30420.1, WP_143713288.1, WP_107417268.1, WP_106966793.1, WP_092287378.1, WP_080048780.1, WP_122982115.1, PWK87198.1, KYG52038.1, OUE26840.1, WP_052685689.1, SBV25448.1, MAM88718.1, RZI82703.1, WP_107115530.1, KJK32958.1, WP_146103553.1, WP_132628273.1, ALD14029.1, RZL00883.1, WP_148029222.1, WP_105645204.1, WP_108710096.1, WP_020639922.1, and WP_132200424.1.

The method of identifying the polypeptide having the PET degradation activity of the present disclosure comprises the steps of:
preparing an amino acid sequence of a polypeptide (predicted polypeptide) predicted to have the PET degradation activity;
aligning the prepared amino acid sequence with the reference sequence of SEQ ID NO: 1; and
determining from the alignment result whether the amino acid sequence of the predicted polypeptide has the following characteristics:
   based on the reference sequence of SEQ ID NO: 1,
   (1) a residue corresponding to position 62 is F, W, or Y;
   (2) a residue corresponding to position 70 is P;
   (3) a residue corresponding to position 81 is I, L, M, or V;
   (4) a residue corresponding to position 85 is P;
   (5) a residue corresponding to position 86 is G;
   (6) a residue corresponding to position 94 is I, L, M, or V;
   (7) a residue corresponding to position 95 is A or S;
   (8) a residue corresponding to position 96 is A or S;
   (9) a residue corresponding to position 98 is G;
   (10) a residue corresponding to position 99 is F, W or Y;
   (11) a residue corresponding to position 100 is V, L, I, or M;
   (12) a residue corresponding to position 101 is V, L, I, or M;
   (13) a residue corresponding to position 113 is D, N, or E;
   (14) a residue corresponding to position 118 is R, Q, or K;
   (15) a residue corresponding to position 135 is Q, R, E, or K;
   (16) a residue corresponding to position 157 is D, N, or E;
   (17) a residue corresponding to position 165 is G;
   (18) a residue corresponding to position 167 is S, A, N, or T;
   (19) a residue corresponding to position 169 is G;
   (20) a residue corresponding to position 170 is G;
   (21) a residue corresponding to position 262 is D, N, or E;
   (22) a residue corresponding to position 304 is H, N, or Y;
   (23) a residue corresponding to position 322 is W, F, or Y; and
   (24) a residue corresponding to position 330 is D, N, or E.

The terms "alignment" and "corresponding" are the same as described above.

In one embodiment, the amino acid sequence of the predicted polypeptide may be one or more, two or more, or multiple. In one embodiment, the method may comprise a step of aligning a plurality of amino acid sequences of the predicted polypeptide.

In any one embodiment of the above-mentioned embodiments, the method of identifying the polypeptide having the PET degradation activity may comprise the step of examining whether the predicted polypeptide comprises motifs of the following Formulae 1 to 5:

(Formula 1) X1-X2-X3-X4-X5-X6-X7 -X8-P:

wherein X1 is F, W, or Y; and
X2 to X8 are arbitrary amino acids or absent;

(Formula 2) X1-X2-X3-X4-P-G:

wherein X1 is I, L, M, or V; and
X2 to X4 are arbitrary amino acids or absent;

(Formula 3) X1-X2-X3-X4-G-X5-X6-X7:

wherein X1 is I, L, M, or V;
X2 is A or S;
X3 is A or S;
X4 is an arbitrary amino acid or absent;
X5 is F, W, or Y;
X6 is V, L, I, or M; and
X7 is V, L, I, or M;

(Formula 4) X1-X2-X3-X4-X5-X6-X7-X8-G-X9-X10-X11-G-G
wherein X1 is D, N, or E;
X2 to X8, X9 and X11 are arbitrary amino acids or absent; and
X10 is S, A, N, or T;

(Formula 5) X1-X2-X3-X4-X5-X6-X7-X8-X9

wherein X1 is W, F, or Y; and
X2 to X8 are arbitrary amino acids or absent; and
X9 is D, N or E.

The polypeptide having the PET degradation activity which is provided in the present disclosure is a polypeptide, wherein
based on the reference sequence of SEQ ID NO: 1,
(1) a residue corresponding to position 62 is F, W, or Y;
(2) a residue corresponding to position 70 is P;
(3) a residue corresponding to position 81 is I, L, M, or V;
(4) a residue corresponding to position 85 is P;
(5) a residue corresponding to position 86 is G;
(6) a residue corresponding to position 94 is I, L, M, or V;
(7) a residue corresponding to position 95 is A or S;
(8) a residue corresponding to position 96 is A or S;
(9) a residue corresponding to position 98 is G;
(10) a residue corresponding to position 99 is F, W or Y;
(11) a residue corresponding to position 100 is V, L, I, or M;
(12) a residue corresponding to position 101 is V, L, I, or M;
(13) a residue corresponding to position 113 is D, N, or E;
(14) a residue corresponding to position 118 is R, Q, or K;
(15) a residue corresponding to position 135 is Q, R, E, or K;
(16) a residue corresponding to position 157 is D, N, or E;
(17) a residue corresponding to position 165 is G;
(18) a residue corresponding to position 167 is S, A, N, or T;
(19) a residue corresponding to position 169 is G;
(20) a residue corresponding to position 170 is G;
(21) a residue corresponding to position 262 is D, N, or E;
(22) a residue corresponding to position 304 is H, N, or Y;
(23) a residue corresponding to position 322 is W, F, or Y; and
(24) a residue corresponding to position 330 is D, N, or E.

In one embodiment, the polypeptide of the present disclosure may comprise the motifs of the following Formulae 1 to 5:

(Formula 1) X1-X2-X3-X4-X5-X6-X7 -X8-P:

wherein X1 is F, W, or Y; and
X2 to X8 are arbitrary amino acids or absent;

(Formula 2) X1-X2-X3-X4-P-G:

wherein X1 is I, L, M, or V; and
X2 to X4 are arbitrary amino acids or absent;

(Formula 3) X1-X2-X3-X4-G-X5-X6-X7:

wherein X1 is I, L, M, or V;
X2 is A or S;
X3 is A or S;
X4 is an arbitrary amino acid or absent;
X5 is F, W, or Y;
X6 is V, L, I, or M; and
X7 is V, L, I, or M;

(Formula 4) X1-X2-X3-X4-X5-X6-X7-X8-G-X9-X10-X11-G-G

wherein X1 is D, N, or E;
X2 to X8, X9 and X11 are arbitrary amino acids or absent; and
X10 is S, A, N, or T;

(Formula 5) X1-X2-X3-X4-X5-X6-X7-X8-X9

wherein X1 is W, F, or Y; and
X2 to X8 are arbitrary amino acids or absent; and
X9 is D, N or E.

In any one embodiment of the above-mentioned embodiments, the polypeptide having the PET degradation activity which is provided in the present disclosure has, comprise, or consists of an amino acid sequence selected from
(i) an amino acid sequence selected from WP_109362551.1, SHM40309.1, SDZ16714.1, WP_084434874.1, WP_101366623.1, WP_117215036.1, WP_132413760.1, WP_110567505.1, WP_143590710.1, WP_091553551.1, WP_124822775.1, WP_116180173.1, OGB27210.1, WP_088988822.1, WP_125191182.1, WP_085749752.1, WP_130464304.1, WP_054022242.1, WP_082377728.1, WP_132386942.1, WP_082403176.1, WP_013736455.1, WP_073887759.1, CCH19814.1, WP_141984372.1, WP_155388305.1, WP_039906502.1, WP_137976057.1, WP_132337032.1, WP_093411427.1, WP_075737041.1, WP_139584963.1, WP_088276085.1, PIG53436.1, WP_045318561.1, WP_078759821.1, AEV21261.1, WP_047194864.1, WP_083950838.1, KWV31814.1, ADV92525.1, EOD65379.1, WP_013229249.1, WP_131056699.1, WP_072476921.1, WP_065917710.1, WP_130401777.1, WP_081971423.1, WP_086849962.1, WP_109639600.1, WP_150303381.1, WP_076469402.1, WP_086784246.1, WP_125313231.1, WP_120675340.1, WP_027346283.1, WP_155541557.1, WP_075975245.1, WP_144126986.1, WP_078844206.1, WP_091283529.1, WP_103503499.1, WP_130969140.1, WP_112669767.1, KNX38636.1, GBD22443.1, WP_083302694.1, WP_091084084.1, WP_093954453.1, WP_116813002.1, WP_089021493.1, PWK39581.1, WP_011291330.1, WP_123685830.1, WP_123748612.1, WP_084400763.1, WP_132183354.1, ADV92527.1, WP_014689902.1, WP _093935592.1, RZS34008.1, WP_138709315.1, WP_141569747.1, 1JFR_A, WP_124770494.1, 4WFI_A, WP_143737449.1, WP_080041749.1, CBY05529.1, WP_111700265.1, OKK16161.1, WP_143320421.1, WP_084578793.1, WP_104613137.1, WP_104115406.1, SPF04022.1, WP_119701604.1, WP_120326365.1, PWW60275.1, WP_086668955.1, WP_092528534.1, WP_063729606.1, 3VIS_A, WP_138698985.1, WP_145923095.1, WP_068752972.1, MBA55398.1, WP_123888242.1, WP_083724990.1, WP_044499735.1, WP_051808300.1, WP_067035690.1, WP_103564314.1, WP_129849678.1, WP_125627453.1, WP_083947829.1, WP_083303042.1, KOV66859.1, SDH02982.1, PKM30861.1, WP_103939557.1, WP_120781673.1, WP_136529585.1, WP_091310325.1, GAP50618.1, SCF11721.1, WP_130477875.1, KWV30420.1, WP_143713288.1, WP_107417268.1, WP_106966793.1, WP_092287378.1, WP_080048780.1, WP_122982115.1, PWK87198.1, KYG52038.1, OUE26840.1, WP_052685689.1, SBV25448.1, MAM88718.1, RZI82703.1, WP_107115530.1, KJK32958.1, WP_146103553.1, WP_132628273.1, ALD14029.1, RZL00883.1, WP_148029222.1, WP_105645204.1, WP_108710096.1, WP_020639922.1, and WP_132200424.1;
(ii) an amino acid sequence having 70% or more identity to the amino acid sequence of (i);
(iii) a polypeptide encoded by a polynucleotide having 70% or more sequence identity to a coding sequence of a mature polypeptide of the amino acid sequence of (i);
(iv) a polypeptide encoded by a polynucleotide that hybridizes with (a) the coding sequence of the mature polypeptide of the amino acid sequence of (i), (b) cDNA thereof, or (c) the full-length complement of (a) or (b) under low stringency conditions, medium stringency conditions, medium-high stringency conditions, high stringency conditions, or very high stringency conditions; and
(v) a functional fragment of any one polypeptide of (i) to (iv) that has the PET degradation activity, and
   the polypeptide may comprise an amino acid sequence, wherein
   based on the reference sequence of SEQ ID NO: 1,
      (1) a residue corresponding to position 62 is F, W, or Y;
      (2) a residue corresponding to position 70 is P;
      (3) a residue corresponding to position 81 is I, L, M, or V;
      (4) a residue corresponding to position 85 is P;
      (5) a residue corresponding to position 86 is G;
      (6) a residue corresponding to position 94 is I, L, M, or V;
      (7) a residue corresponding to position 95 is A or S;
      (8) a residue corresponding to position 96 is A or S;
      (9) a residue corresponding to position 98 is G;
      (10) a residue corresponding to position 99 is F, W or Y;
      (11) a residue corresponding to position 100 is V, L, I, or M;
      (12) a residue corresponding to position 101 is V, L, I, or M;
      (13) a residue corresponding to position 113 is D, N, or E;
      (14) a residue corresponding to position 118 is R, Q, or K;
      (15) a residue corresponding to position 135 is Q, R, E, or K;
      (16) a residue corresponding to position 157 is D, N, or E;
      (17) a residue corresponding to position 165 is G;
      (18) a residue corresponding to position 167 is S, A, N, or T;
      (19) a residue corresponding to position 169 is G;
      (20) a residue corresponding to position 170 is G;
      (21) a residue corresponding to position 262 is D, N, or E;
      (22) a residue corresponding to position 304 is H, N, or Y;
      (23) a residue corresponding to position 322 is W, F, or Y; and
      (24) a residue corresponding to position 330 is D, N, or E, and
   as long as the polypeptide has the activity identical or corresponding to that of the polypeptide of (i), it may be comprised without limitation in the scope of the polypeptide having the PET degradation activity of the present disclosure.

In any one embodiment of the above-mentioned embodiments, the polypeptide having the PET degradation activity has, comprises, or consists of an amino acid sequence selected from
(i) an amino acid sequence selected from SDZ16714.1, SHM40309.1, WP_117215036.1, WP_084434874.1, WP_116180173.1, WP_109362551.1, WP_091553551.1, WP_132413760.1, OGB27210.1, WP_101366623.1, WP_124822775.1, WP_110567505.1, WP_054022242.1, WP_130464304.1, WP_073887759.1, WP_088988822.1, WP_082377728.1, WP_132386942.1, CCH19814.1, WP_039906502.1, WP_132337032.1 and PIG53436.1, WP_082403176.1, WP_137976057.1, WP_085749752.1, WP_125191182.1, WP_141984372.1, WP_155388305.1, WP_013736455.1, WP_143590710.1, WP_139584963.1, WP_078759821.1, AEV21261.1, WP_083950838.1, KWV31814.1, ADV92525.1, and OKK16161.1; (ii) an amino acid sequence having 70% or more identity to the amino acid sequence of (i); (iii) a polypeptide encoded by a polynucleotide having 70% or more sequence identity to a coding sequence of a mature polypeptide of the amino acid sequence of (i); (iv) a polypeptide encoded by a polynucleotide that hybridizes with (a) the coding sequence of the mature polypeptide of the amino acid sequence of (i), (b) cDNA thereof, or (c) the full-length complement of (a) or (b) under low stringency conditions, medium stringency conditions, medium-high stringency conditions, high stringency conditions, or very high stringency conditions; and (v) a functional fragment of any one polypeptide of (i) to (iv) that has the PET degradation activity, and
based on the reference sequence of SEQ ID NO: 1,
   (1) a residue corresponding to position 62 is F, W, or Y;
   (2) a residue corresponding to position 70 is P;
   (3) a residue corresponding to position 81 is I, L, M, or V;
   (4) a residue corresponding to position 85 is P;
   (5) a residue corresponding to position 86 is G;
   (6) a residue corresponding to position 94 is I, L, M, or V;
   (7) a residue corresponding to position 95 is A or S;
   (8) a residue corresponding to position 96 is A or S;
   (9) a residue corresponding to position 98 is G;
   (10) a residue corresponding to position 99 is F, W or Y;
   (11) a residue corresponding to position 100 is V, L, I, or M;
   (12) a residue corresponding to position 101 is V, L, I, or M;
   (13) a residue corresponding to position 113 is D, N, or E;
   (14) a residue corresponding to position 118 is R, Q, or K;
   (15) a residue corresponding to position 135 is Q, R, E, or K;
   (16) a residue corresponding to position 157 is D, N, or E;
   (17) a residue corresponding to position 165 is G;
   (18) a residue corresponding to position 167 is S, A, N, or T;
   (19) a residue corresponding to position 169 is G;
   (20) a residue corresponding to position 170 is G;
   (21) a residue corresponding to position 262 is D, N, or E;
   (22) a residue corresponding to position 304 is H, N, or Y;
   (23) a residue corresponding to position 322 is W, F, or Y; and
   (24) a residue corresponding to position 330 is D, N, or E.

In any one embodiment of the above-mentioned embodiments, the polypeptide having the PET degradation activity has, comprise, or consists of an amino acid sequence selected from
(i) an amino acid sequence selected from SDZ16714.1, SHM40309.1, WP_117215036.1, WP_084434874.1, WP_116180173.1, WP_109362551.1, WP_091553551.1, WP_132413760.1, OGB27210.1, WP_101366623.1, WP_124822775.1, WP_110567505.1, WP_054022242.1, WP_130464304.1, WP_073887759.1, WP_088988822.1, WP_082377728.1, WP_132386942.1, CCH19814.1, WP_039906502.1, WP_132337032.1, and PIG53436.1; (ii) an amino acid sequence having 70% or more identity to the amino acid sequence of (i); (iii) a polypeptide encoded by a polynucleotide having 70% or more sequence identity to a coding sequence of a mature polypeptide of the amino acid sequence of (i); (iv) a polypeptide encoded by a polynucleotide that hybridizes with (a) the coding sequence of the mature polypeptide of the amino acid sequence of (i), (b) cDNA thereof, or (c) the full-length complement of (a) or (b) under low stringency conditions, medium stringency conditions, medium-high stringency conditions, high stringency conditions, or very high stringency conditions; and (v) a functional fragment of any one polypeptide of (i) to (iv) that has the PET degradation activity, and
based on the reference sequence of SEQ ID NO: 1,
   (1) a residue corresponding to position 62 is F, W, or Y;
   (2) a residue corresponding to position 70 is P;
   (3) a residue corresponding to position 81 is I, L, M, or V;
   (4) a residue corresponding to position 85 is P;
   (5) a residue corresponding to position 86 is G;
   (6) a residue corresponding to position 94 is I, L, M, or V;
   (7) a residue corresponding to position 95 is A or S;
   (8) a residue corresponding to position 96 is A or S;
   (9) a residue corresponding to position 98 is G;
   (10) a residue corresponding to position 99 is F, W or Y;
   (11) a residue corresponding to position 100 is V, L, I, or M;
   (12) a residue corresponding to position 101 is V, L, I, or M;
   (13) a residue corresponding to position 113 is D, N, or E;
   (14) a residue corresponding to position 118 is R, Q, or K;
   (15) a residue corresponding to position 135 is Q, R, E, or K;
   (16) a residue corresponding to position 157 is D, N, or E;
   (17) a residue corresponding to position 165 is G;
   (18) a residue corresponding to position 167 is S, A, N, or T;
   (19) a residue corresponding to position 169 is G;
   (20) a residue corresponding to position 170 is G;
   (21) a residue corresponding to position 262 is D, N, or E;
   (22) a residue corresponding to position 304 is H, N, or Y;
   (23) a residue corresponding to position 322 is W, F, or Y; and
   (24) a residue corresponding to position 330 is D, N, or E.

In any one embodiment of the above-mentioned embodiments, the polypeptide having the PET degradation activity may have 80% or more sequence identity or homology to WP_039906502.1. For example, it may be selected from CCH19814.1, WP_110567505.1, WP_112669767.1, WP_124822775.1, WP_089021493.1, WP_139584963.1, WP_130401777.1, PIG53436.1, WP_088988822.1, WP_082377728.1, WP_091553551.1, WP_101366623.1, WP_124770494.1, KWV31814.1, WP_083302694.1, WP_091283529.1, WP_093411427.1, WP_131056699.1, WP_013736455.1, and WP_130464304.1.

As used herein, the terms "polypeptide having PET degradation activity" and "PETase" refer to an enzyme having depolymerization activity for polyethylene terephthalate (PET). For example, the PETase may have depolymerization activity for PET, as well as depolymerization activity for an oligomer obtained by depolymerization thereof, e.g., for bis(2-hydroxyethyl) terephthalate (BHET). For another example, the PETase may have degradation activity for polyester bonds.

In the present disclosure, the PET degradation activity may be measured and evaluated using methods known in the art by comprising the embodiments described herein, for example, may be evaluated by measuring the production amount of BHET, MHET, TPA, or EG.

For example, the polypeptide having PET degradation activity of the present disclosure may be an enzyme derived from a microorganism or a recombinant enzyme.

Another aspect of the present disclosure provides use of the polypeptide as a PETase.

The "polynucleotide" of the present disclosure may comprise the coding sequence of the above-described polypeptide. The polynucleotide may undergo various modifications in the coding region within the scope that does not change the amino acid sequence of the polypeptide, due to codon degeneracy or in consideration of the codons preferred in an organism in which the polypeptide is to be expressed.

Further, the polynucleotide of the present disclosure may comprise a probe that may be prepared from a known gene sequence, for example, any sequence encoding the polypeptide of the present disclosure by hybridizing with a sequence complementary to all or part of the nucleotide sequence under stringent conditions without limitation.

The "stringent conditions" refers to conditions under which specific hybridization between polynucleotides is allowed. Such conditions are specifically described in the literature (e.g., J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York).

For example, the stringent conditions may comprise conditions under which polynucleotides having a high homology or identity of 40% or more, specifically 90% or more, more specifically 95% or more, 96% or more, 97% or more, 98% or more, and even more specifically 99% or more are hybridized with each other and polynucleotides having a homology or identity lower than the above homologies or identities are not hybridized with each other, or washing conditions of the common Southern hybridization, washing once, or specifically, twice or three times at a salt concentration and a temperature corresponding to 60°C, 1×SSC, 0.1% SDS, specifically, 60°C, 0.1×SSC, 0.1% SDS, and more specifically, 68°C, 0.1×SSC, 0.1% SDS.

Hybridization requires that two nucleic acids have complementary sequences, although mismatches between bases are possible depending on the stringency of the hybridization. The term "complementary" is used to describe the relationship between nucleotide bases that may hybridize with each other. For example, with respect to DNA, adenosine is complementary to thymine, and cytosine is complementary to guanine. Therefore, the polynucleotide of the present disclosure may comprise isolated nucleotide fragments complementary to the entire sequence as well as nucleic acid sequences substantially similar thereto.

Specifically, polynucleotides having a homology or identity may be detected using the hybridization conditions comprising a hybridization step at a Tm value of 55°C under the above-described conditions. Further, the Tm value may be 60°C, 63°C, or 65°C, but is not limited thereto, and may be appropriately adjusted by those skilled in the art depending on the purpose thereof.

The appropriate stringency for hybridizing the polynucleotides depends on the length of the polynucleotides and the degree of complementation, and these variables are well known in the art (see, Sambrook et al., supra, 9.50-9.51, 11.7-11.8).

For example, the "high stringency" may occur at about 5°C to 10°C below the Tm of the probe; the "medium stringency" may occur at about 10°C to 20°C below the Tm of the probe; and the "low stringency" may occur at about 20°C to 25°C below the Tm, but the stringency is not limited thereto.

For example, the "low stringency condition" may mean, for probes of at least about 100 nucleotides in length, prehybridization and hybridization at 42°C in 5×SSPE, 0.3% SDS, 200 micrograms/ml of sheared and denatured salmon sperm DNA, and 25% formamide, according to the standard Southern blotting procedures for 12 hours to 24 hours. The carrier material may be finally washed twice or three times each for 15 minutes in 2 X SSC, 0.1% to 0.2% SDS at 50°C.

For example, the "medium stringency condition" may mean, for probes of at least about 100 nucleotides in length, prehybridization and hybridization at 42°C in 5×SSPE, 0.3% SDS, 200 micrograms/ml of sheared and denatured salmon sperm DNA, and 35% formamide, according to the standard Southern blotting procedures for 12 hours to 24 hours. The carrier material may be finally washed twice or three times each for 15 minutes in 2 X SSC, 0.1% to 0.2% SDS at 55°C. For example, the "medium-high stringency condition" may mean, for probes of at least about 100 nucleotides in length, prehybridization and hybridization at 42°C in 5×SSPE, 0.3% SDS, 200 micrograms/ml of sheared and denatured salmon sperm DNA, and 35% formamide, according to the standard Southern blotting procedures for 12 hours to 24 hours. The carrier material may be finally washed twice or three times each for 15 minutes in 1 X to 2 X SSC, 0.1% to 0.2% SDS at 60°C.

For example, the "high stringency condition" may mean, for probes of at least about 100 nucleotides in length, prehybridization and hybridization at 42°C in 5×SSPE, 0.3% SDS, 200 micrograms/ml of sheared and denatured salmon sperm DNA, and 35% formamide, according to the standard Southern blotting procedures for 12 hours to 24 hours. The carrier material may be finally washed twice or three times each for 15 minutes in 2 X SSC, 0.1% to 0.2% SDS at 65°C.

The "nucleic acid construct" provided in the present disclosure comprises a polynucleotide encoding the polypeptide provided in the present disclosure, which is operably linked to one or more regulatory sequences that direct expression of the coding sequence in a suitable host cell under conditions suitable for the regulatory sequences.

Polynucleotides may be manipulated in a variety of ways to allow expression of the polypeptide. Depending on the expression vector, it may be desirable or necessary to manipulate the polynucleotides prior to insertion into the vector. Such manipulations may be performed using methods known in the art.

The "vector" provided in the present disclosure refers to a DNA construct comprising the nucleotide sequence of the polynucleotide encoding the polypeptide operably linked to a suitable expression regulatory region (expression regulatory sequence) so as to be able to express the polypeptide of the present disclosure in a suitable host cell. The expression regulatory region may comprise a promoter capable of initiating transcription, any operator sequence for regulating the transcription, a sequence encoding a suitable mRNA ribosome-binding site, and a sequence for regulating termination of transcription and translation. Once transformed into a suitable host cell, the vector may replicate or function independently from the host genome, or may integrate into the genome thereof.

The vector that may be used in the present disclosure is not particularly limited, and any vector known in the art may be used. Examples of the vectors commonly used may comprise natural or recombinant plasmids, cosmids, viruses, and bacteriophages. For example, as a phage vector or cosmid vector, pWE15, M13, MBL3, MBL4, IXII,ASHII, APII, t10, t11, Charon4A, and Charon21A, etc. may be used, and as a plasmid vector, those based on pBR, pUC, pBluescriptll, pGEM, pTZ, pCL and pET, etc. may be used. Specifically, pDZ, pACYC177, pACYC184, pCL, pECCG117, pUC19, pBR322, pMW118, pCC1BAC vector, etc. may be used.

For example, the polynucleotide encoding the polypeptide provided in the present disclosure may be inserted into the chromosome through a vector for intracellular chromosomal insertion. The insertion of the polynucleotide into the chromosome may be performed by any method known in the art, for example, by homologous recombination, but is not limited thereto. The vector may further comprise a selection marker to confirm the insertion into the chromosome. The selection marker is for selecting the cells transformed with the vector, that is, for confirming whether the target nucleic acid molecule has been inserted, and markers that provide selectable phenotypes, such as drug resistance, auxotrophy, resistance to cytotoxic agents, or expression of surface polypeptides, may be used. Only cells expressing the selection marker are able to survive or show different phenotypes under the environment treated with the selective agent, and thus the transformed cells may be selected.

The "host cell" of the present disclosure may comprise any host cell without limitation as long as it is able to express the polypeptide of the present disclosure.

The host cell of the present disclosure may comprise the above-described polypeptide, the polynucleotide encoding the polypeptide, the nucleic acid construct and/or vector comprising the same.

The nucleic acid construct or vector may be integrated into the chromosome as described earlier, or may remain as a self-replicating extrachromosomal vector.

The host cell of the present disclosure encompasses any progeny of a parent cell that is not identical to the parent cell due to mutations that occur during replication.

The host cell may be any cell useful in the recombinant production of the polypeptide, e.g., a prokaryotic cell or a eukaryotic cell.

The prokaryotic host cell may be any Gram-positive or Gram-negative bacterium.

The Gram-positive bacteria comprise, but are not limited to, *Bacillus, Clostridium, Enterococcus, Geobacillus, Lactobacillus, Lactococcus, Oceanobacillus, Staphylococcus, Streptococcus, Corynebacterium,* and *Streptomyces.*

The Gram-negative bacteria comprise, but are not limited to, *Campylobacter, Escherichia, Flavobacterium, Fusobacterium, Helicobacter, Ilyobacter, Neisseria, Pseudomonas, Salmonella, Vibrio* (e.g., *Vibrio natriegens*), and *Ureaplasma.*

In one specific embodiment, the bacterial host cell may be a host cell belonging to the genus *Bacillus,* specifically, comprising *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* and *Bacillus thuringiensis* cells, but is not limited thereto.

In one specific embodiment, the bacterial host cell may be a host cell belonging to the genus *Streptococcus,* specifically, comprising *Streptococcus equisimilis, Streptococcus pyogenes, Streptococcus uberis,* and *Streptococcus equi subsp. Zooepidemicus* cells, but is not limited thereto.

In one specific embodiment, the bacterial host cell may be a host cell belonging to the genus *Streptomyces,* specifically, comprising *Streptomyces achromogenes, Streptomyces avermitilis, Streptomyces coelicolor, Streptomyces griseus,* and *Streptomyces lividans* cells, but is not limited thereto.

In one specific embodiment, the bacterial host cell may be a host cell belonging to the genus *Corynebacterium,* specifically, *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens,* but is not limited thereto.

In one specific embodiment, the bacterial host cell may be a host cell of the genus *Escherichia,* and may be *Escherichia coli* (*E.coli*), but is not limited thereto.

The host cell may be a eukaryotic cell, such as a mammalian, insect, plant, or fungal cell.

The host cell may be a fungal cell. As used herein, the "fungi" may comprise *Ascomycota, Basidiomycota, Chytridiomycota,* and *Zygomycota* as well as the *Oomycota* and all Fungi *imperfecti.*

The fungal host cell may be a yeast cell. As used herein, the "yeast" comprises *ascosporogenous* yeast (*Endomycetales*), *basidiosporogenous* yeast, and yeast belonging to the Fungi *imperfecti* (*Blastomycetes*). However, this classification may change, and may be defined as described in Biology and Activities of Yeast (Skinner, Passmore, and Davenport, editors, Soc. App. Bacteriol. Symposium Series No. 9, 1980).

The yeast host cell may be a *Candida, Hansenula, Kluyveromyces, Pichia, Komagataella, Saccharomyces, Schizosaccharomyces,* or *Yarrowia* cell, for example, *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis, Komagataella phaffii,* or *Yarrowia lipolytica* cell.

The fungal host cell may be a filamentous fungal cell. The "filamentous fungi" comprise all filamentous forms of the subdivision *Eumycota* and *Oomycota* (as defined by the above literature (Hawksworth et al., 1995)). The filamentous fungi are generally characterized by a mycelial wall composed of chitin, cellulose, glucan, chitosan, mannan, and other complex polysaccharides. Vegetative growth is by hyphal elongation, and carbon catabolism is obligately aerobic. In contrast, vegetative growth by yeasts, for example, *Saccharomyces cerevisiae* is by budding of a unicellular thallus, and carbon catabolism may be fermentative.

The filamentous fungal host cell may be an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell.

For example, the filamentous fungal host cell may be an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell, but is not limited thereto.

The "composition" of the present disclosure may comprise the polypeptide having the PET degradation activity or the host cell expressing the polypeptide.

The descriptions of the polypeptide having the PET degradation activity provided in the present disclosure and the host cell expressing the same may be applied to the polypeptide having the PET degradation activity or the host cell expressing the polypeptide.

The composition of the present disclosure may be used in converting polyester into a final product.

The term "polyester" refers to a polymer comprising an ester functional group in the main chain of its structure. For example, polyethylene terephthalate is a semi-aromatic copolymer composed of two monomers, terephthalic acid and ethylene glycol (EG).

The polyester may be selected from polyethylene terephthalate (PET), polyethylene terephthalate glycol (PETG), polyethylene co-isosorbide-terephthalate (PEIT), polytrimethylene terephthalate (PTT), polybutylene adipate terephthalate (PBAT), polycyclohexylenedimethylene terephthalate (PCT), and polybutylene terephthalate (PBT). Specifically, the polyester may be PET.

The polypeptide of the present disclosure or the composition comprising the same may be used to depolymerize PET into bis(2-hydroxyethyl) terephthalate (BHET), or to decompose PET into mono(2-hydroxymethyl) terephthalate (MHET) and terephthalic acid (TPA).

The polypeptide of the present disclosure or the composition comprising the same may be used to degrade polymers (e.g., oligomers) resulting from PET depolymerization. For example, it may be used to degrade BHET into MHET and TPA.

The composition of the present disclosure may further comprise other components, in addition to the polypeptide having the PET degradation activity, which is provided in the present disclosure. The components added to the composition of the present disclosure may be appropriately selected by those skilled in the art.

In one specific embodiment, the composition of the present disclosure may further comprise any components suitable for being applied to conversion of PET into the final products.

In one specific embodiment, the composition of the present disclosure may further comprise any components suitable for being applied to PET degradation.

Examples of materials that may be added comprise stabilizers, surfactants, builders, chelating agents, dispersants, enzymes, enzyme stabilizers, catalysts, activators, carriers, binders, lubricants, disintegrants, excipients, solubilizers, suspending agents, colorants, flavoring agents, buffers, preservatives, analgesics, solubilizers, isotonic agents, stabilizers, diluents, lubricants, preservatives, etc., but are not limited thereto.

In one specific embodiment, the composition provided in the present disclosure may further comprise a naturally occurring material or a non-naturally occurring material, in addition to the polypeptide provided in the present disclosure.

In one specific embodiment, the composition provided in the present disclosure may further comprise additional enzymes, in addition to the polypeptide provided in the present disclosure.

A method of preparing the polypeptide of the present disclosure may comprise the steps of culturing the host ell; and recovering the polypeptide expressed in the culturing step.

As used herein, the term "culturing" means that the host cell is grown under appropriately controlled environmental conditions. The culturing process of the present disclosure may be performed in a suitable medium and culture conditions known in the art. Such a culturing process may be easily adjusted for use by those skilled in the art according to the strain to be selected. Specifically, the culture may be a batch culture, a continuous culture, and a fed-batch culture, but is not limited thereto.

As used herein, the term "medium" refers to a mixture of materials which comprise, as a main ingredient, nutrient materials required for culturing the host cell, and it supplies nutrient materials and growth factors, along with water that is essential for survival and growth. Specifically, the medium and other culture conditions used for culturing the host cell of the present disclosure may be any medium commonly used for culturing host cells without any particular limitation. However, the host cell of the present disclosure may be cultured under aerobic conditions in a common medium comprising appropriate carbon sources, nitrogen sources, phosphorus sources, inorganic compounds, amino acids, and/or vitamins, etc., while adjusting temperature, pH, etc.

In the present disclosure, the carbon sources may comprise carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; amino acids, such as glutamic acid, methionine, lysine, etc. Further, the carbon sources may comprise natural organic nutrients such as starch hydrolysate, molasses, blackstrap molasses, rice bran, cassava, sugar cane molasses, and corn steep liquor, etc. Specifically, carbohydrates such as glucose and sterilized pretreated molasses (i.e., molasses converted to reducing sugar) may be used, and in addition, various other carbon sources may be used in an appropriate amount without limitation. These carbon sources may be used alone or in combination of two or more thereof, but are not limited thereto.

The nitrogen sources may comprise inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extract, yeast extract, malt extract, corn steep liquor, casein hydrolysate, fish or decomposition product thereof, defatted soybean cake or decomposition product thereof, etc. These nitrogen sources may be used alone or in combination of two or more thereof, but are not limited thereto.

The phosphorus sources may comprise monopotassium phosphate, dipotassium phosphate, or corresponding sodium-comprising salts, etc. The inorganic compounds may comprise sodium chloride, calcium chloride, iron chloride, magnesium sulfate, iron sulfate, manganese sulfate, calcium carbonate, etc. In addition, amino acids, vitamins, and/or appropriate precursors, etc. may be comprised. These components or precursors may be added to a medium in a batch or continuous manner, but these phosphorus sources are not limited thereto.

Further, pH of the medium may be adjusted by adding, to the medium, a compound such as ammonium hydroxide, potassium hydroxide, ammonia, phosphoric acid, sulfuric acid, etc. in an appropriate manner during the culturing of the host cell. In addition, bubble formation may be prevented during the culturing using an antifoaming agent such as fatty acid polyglycol ester. Further, oxygen or a gas comprising oxygen may be injected to the medium to maintain aerobic conditions of the medium, or no gas may be injected or nitrogen, hydrogen, or carbon dioxide gas may be injected to maintain anaerobic or microaerobic conditions, but are not limited thereto.

The temperature of the medium may be 20°C to 50°C, specifically, 25°C to 40°C, but is not limited thereto. The culturing may be continued until the desired amount of a useful material is obtained, and may be specifically carried out for 24 hours to 196 hours, but is not limited thereto.

In one specific embodiment, the polypeptide expressed in the culturing step may be recovered using methods known in the art to which the present disclosure pertains. For example, the polypeptide may be recovered from a nutrient medium by common procedures comprising, but are not limited to, collection, centrifugation, filtration, extraction, spray-drying, evaporation, or precipitation.

The recovering method is to collect the polypeptide using the method of culturing the host cell of the present disclosure, for example, using a suitable method known in the art according to a batch culture, continuous culture, or fed-batch culture method. For example, methods such as centrifugation, filtration, treatment with a protein crystallizing precipitant (salting-out method), extraction, ultrasonic disruption, ultrafiltration, dialysis, various kinds of chromatographies such as molecular sieve chromatography (gel filtration), adsorption chromatography, ion exchange chromatography, affinity chromatography, etc., HPLC, and a combination thereof may be used, and the polypeptide may be recovered from the medium or the host cell using suitable methods known in the art.

In another specific embodiment, the polypeptide expressed by the host cell in the culturing step may not be recovered. In the specific embodiment, the host cell expressing the polypeptide may be used as it is as a source of the polypeptide.

The present disclosure may comprise a method of producing mono(2-hydroxymethyl) terephthalate (MHET), terephthalic acid (TPA), and/or ethylene glycol (EG), the method comprising bringing a polyester into contact with the above-described polypeptide having the PET degradation activity or the host cell expressing the polypeptide, or the composition comprising the same.

Specifically, the polyester may comprise the aforementioned descriptions.

In the present disclosure, the polyester may be degraded by bringing the polyester into "contact" with the polypeptide having the PET degradation activity or the host cell expressing the polypeptide or the composition comprising the same, but is not limited thereto. The degradation may also be referred to as depolymerization.

The time required for degradation of the polyester may vary depending on the polyester-comprising article itself (i.e., nature and origin of the plastic product, its composition, shape etc.), the type and amount of the polypeptide having the PET degradation activity to be used, as well as various process parameters (i.e., temperature, pH, additional agents, etc.). The process parameters may be easily applied to the polyester-comprising article using a technology known in the art.

For example, the degradation process may be carried out at 20°C to 90°C, preferably at 40°C to 80°C, and more preferably at 50°C to 70°C. More specifically, the temperature may be maintained below an inactivation temperature, which corresponds to the temperature at which the polypeptide becomes inactive and/or below a temperature at which the host cell no longer synthesizes the polypeptide.

For example, the degradation process may be carried out at pH 5 to pH 11, preferably at pH 6 to pH 9.

In the present disclosure, in a specific embodiment, the polyester-comprising article may be pretreated prior to being brought into contact with the polypeptide having the PET degradation activity of the present disclosure to physically or chemically change its structure, thereby increasing the contact area with the polypeptide.

Specifically, MHET, TPA, and/or EG resulting from the degradation by the contact may be recovered, sequentially or continuously.

Specifically, the recovered MHET, TPA, and/or EG may be further purified using all suitable purification method and may be produced in a re-polymerizable form. More preferably, the purification may comprise a stripping process, separation by an aqueous solution, steam selective condensation, filtration and concentration of the medium after the bioprocess, separation, distillation, vacuum evaporation, extraction, electrodialysis, adsorption, ion exchange, precipitation, crystallization, concentration and acid addition dehydration and precipitation, nanofiltration, acid catalyst treatment, semi continuous mode distillation or continuous mode distillation, solvent extraction, evaporative concentration, evaporative crystallization, liquid/liquid extraction, hydrogenation, an azeotropic distillation process, adsorption, column chromatography, simple vacuum distillation and microfiltration, but is not limited thereto.

The final products MHET, TPA, and/or EG obtained by using the composition of the present disclosure may be recycled in the polymerization of polyesters.

Specifically, the obtained final products, MHET, TPA, and/or EG may be recycled as repolymerizable monomers and/or oligomers to synthesize polyester. Specifically, polyesters with the same properties may be repolymerized and mixed with another monomer and/or oligomer to synthesize, for example, new copolymers.

The polyester may be selected from polyethylene terephthalate (PET), polyethylene terephthalate glycol (PETG), polyethylene co-isosorbide-terephthalate (PEIT), polytrimethylene terephthalate (PTT), polybutylene adipate terephthalate (PBAT), polycyclohexylenedimethylene terephthalate (PCT), and polybutylene terephthalate (PBT). Specifically, the polyester may be PET.

The method of synthesizing the polyester using MHET, TPA, and/or EG is known in the art.

### Example

Hereinafter, the present disclosure will be described in more detail with reference to Examples and Experimental Examples. However, these Examples and Experimental Examples are only for illustrating the present disclosure, and the scope of the present disclosure is not intended to be limited by these Examples and Experimental Examples.

### Example 1. Identification of PETase Candidates from NCBI Database

In order to find novel enzymes with PET degradation activity, 20 seeds known to have PET degradation activity were searched using PSI-BLAST with default parameters in Genbank Release 235 (Dec 15 2019, https://www.ncbi.nlm.nih.gov/genbank/release/) and RefSeq-based non-redundant databases, and the resulting sequence lists were merged. The above 20 seed sequences are summarized and shown in Table 1.

**[Table 1]**

| Seed sequence | Genbank | PSI-BLAST cutoff | Hit seqs |
|---|---|---|---|
| Thh Est | AFA45122 | 1.00E-40 | 1804 |
| Est1 | BA199230 | 1.00E-23 | 2082 |
| Est2 | BAK48590 | 1.00E-40 | 1891 |
| The Cut2 | ADV92527 | 7.00E-25 | 2008 |
| TfCut2 | CBY05530 | 2.00E-22 | 2080 |
| The Cut1 | ADV92526 | 4.00E-37 | 1936 |
| Tfu 0882 | AAZ54920 | 5.00E-24 | 2044 |
| Thf42 Cut1 | ADV92528 | 3.00E-34 | 1911 |
| TfCut1 | CBY05529 | 6.00E-36 | 1926 |
| TfH | AAZ54921 | 5.00E-42 | 1858 |
| BTA2 | CAH17554 | 5.00E-33 | 1961 |
| Cut190 | SFP43695 | 2.00E-33 | 1950 |
| Tcur0390 | ACY95991 | 1.00E-30 | 1956 |
| Tcur1278 | ACY96861 | 1.00E-29 | 1953 |
| LCC | AEV21261 | 5.00E-34 | 1894 |
| PETase | GAP38373 | 2.00E-35 | 1924 |
| PET12 | AKJ29164 | 3.00E-36 | 1939 |
| PET6 | WP 021018894 | 1.00E-30 | 1874 |
| PET5 | CCK74972 | 1.00E-28 | 1961 |
| LIPLAF5-2 | ACC95208 | 3.00E-34 | 1912 |

Among 2103 sequences obtained by the above method, 39 sequences considered to be partial genes (incomplete sequences) were excluded, and a total of 2064 sequences were obtained. A k-tuple pairwise distance (Wilbur and Lipman, 0-1) matrix was generated, and a sequence similarity network was generated through a 0.3 distance cutoff (Wilbur WJ, Lipman DJ. Rapid similarity searches of nucleic acid and protein data banks. Proc Natl Acad Sci USA. 1983 Feb;80(3):726-30.). A sequence representing each cluster was obtained, and a total of 152 sequences were finally obtained. The actual PET degradation activity and strength of the above enzymes were examined in Examples 2 and 3.

### Example 2. Preparation of Enzymes for Analysis of Plastic Degradation Activity

In order to examine the PET degradation activity of the PETase candidates identified in Example 1, the following experiment was conducted. 152 genes of Example 1 which were codon-optimized for *E*. *coli* were synthesized, and amplified by polymerase chain reaction (PCR). A nucleotide sequence corresponding to the signal peptide was removed from each synthesized DNA. Each PCR product was then subcloned into pET22b(+) (Novagen) vector which lacks its own signal peptide. The resulting expression vector pET22b(+) was used to transform *E*. *coli* BL21(DE3)-T1R strain. The *E*. *coli* strain was cultured in a flask comprising 1 L of a lysogeny broth medium supplemented with 200 mg/L ampicillin at 37°C to an optical density at 600 nm of 0.6. Protein expression was induced by adding 0.1 mM isopropyl β-D-1-thiogalactopyranoside (IPTG), and the culture medium was further cultured at 18°C for 16 hours. Subsequently, the cells were harvested by centrifugation at 4000 ×g for 20 minutes at 4°C. The cell pellet was resuspended in buffer A (50 mM Na₂HPO₄-HCl, pH 7.0) and disrupted by sonication. Cell debris was removed by centrifugation at 13500 ×g for 25 minutes, and the supernatant was applied to a Ni-NTA agarose column (Qiagen). After washing with buffer A comprising 30 mM imidazole, the bound protein was eluted with 300 mM imidazole in buffer A. Finally, trace contaminants were removed by size-exclusion chromatography using a Superdex 200 prepgrade column (320 ml, GE Healthcare) equilibrated with buffer A. All purification steps were performed at 4°C. The purity of the protein was examined by sodium dodecyl sulfate polyacrylamide gel electrophoresis. The purified protein was concentrated in 50 mM Na₂HPO₄-HCl (pH 7.0) and 100 mM NaCl.

### Example 3. Analysis of PET Degradation Activity of Prepared PETase Candidates

To analyze the PET degradation activity of the PETase candidates prepared in Example 2, 15 mg of PET bottle powder (B-PET) was prepared and immersed in 1 mL of 50 mM glycine-NaOH (pH 9.0) buffer, together with 500 nM enzyme. B-PET (PET sample derived from PET bottles) was obtained through the following processing. Transparent PET bottles were crushed using a crusher, and then the crushed PET was melted in a high-temperature oven at 270°C. The molten PET was immediately immersed in 4°C water to solidify. The quenched PET thus obtained went through a cryogenic grinding process, and PET powder of 300 µm or less was obtained through a steel mesh. The reaction mixture was reacted at 30°C for 12 hours. Then, the reaction mixture was analyzed using HPLC. After the reaction, the produced MHET and TPA were analyzed through HPLC to evaluate the PET degradation activity. As a control group, the buffer of Example 2 used for enzyme purification was added and reacted, and the PET degradation activities of 152 enzymes as experimental groups were evaluated, which are shown in Table 2.

**[Table 2]**

| Accession code | Concentration of PET degradation product (uM) | Accession code | Concentration of PET degradation product (uM) |
|---|---|---|---|
| Control group | 0.0 | WP 084400763.1 | 46.3 |
| SDZ16714.1 | 1506.2 | ADV92527.1 | 44.1 |
| SHM40309.1 | 991.7 | WP 014689902.1 | 43.8 |
| WP 117215036.1 | 953.4 | WP 093935592.1 | 38.3 |
| WP 084434874.1 | 739.2 | RZS34008.1 | 36.3 |
| WP 116180173.1 | 703.5 | WP 138709315.1 | 34.4 |
| WP 109362551.1 | 694.3 | WP 141569747.1 | 34.0 |
| WP 091553551.1 | 627.5 | 1JFR A | 33.9 |
| WP 132413760.1 | 614.4 | WP 124770494.1 | 31.5 |
| OGB27210.1 | 610.9 | 4WFI A | 30.5 |
| WP_101366623.1 | 593.3 | WP 143737449.1 | 28.7 |
| WP_124822775.1 | 577.6 | CBY05529.1 | 25.7 |
| WP_110567505.1 | 548.1 | WP 111700265.1 | 24.4 |
| WP_054022242.1 | 525.3 | WP 143320421.1 | 23.7 |
| WP_130464304.1 | 483.4 | WP 084578793.1 | 23.0 |
| WP_073887759.1 | 466.6 | WP 104613137.1 | 22.4 |
| WP_088988822.1 | 463.2 | WP 104115406.1 | 20.1 |
| WP 082377728.1 | 432.6 | SPF04022.1 | 19.1 |
| WP 132386942.1 | 432.3 | WP 119701604.1 | 19.0 |
| CCH19814.1 | 426.5 | WP 120326365.1 | 16.7 |
| WP_039906502.1 | 426.5 | PWW60275.1 | 15.5 |
| WP_132337032.1 | 399.5 | WP 086668955.1 | 15.3 |
| PIG53436.1 | 385.4 | WP 092528534.1 | 14.3 |
| WP 082403176.1 | 358.6 | WP 150303381.1 | 13.2 |
| WP 137976057.1 | 355.5 | WP 063729606.1 | 11.8 |
| WP 085749752.1 | 342.4 | 3VIS A | 10.8 |
| WP 125191182.1 | 337.2 | WP 138698985.1 | 10.5 |
| WP 141984372.1 | 333.8 | WP 145923095.1 | 10.0 |
| WP 155388305.1 | 310.5 | WP 068752972.1 | 9.6 |
| WP 013736455.1 | 291.3 | MBA55398.1 | 9.4 |
| WP 143590710.1 | 287.9 | WP 123888242.1 | 8.8 |
| WP 139584963.1 | 271.5 | WP 088276085.1 | 8.7 |
| WP 078759821.1 | 269.2 | WP 083724990.1 | 8.6 |
| AEV21261.1 | 262.5 | WP 044499735.1 | 8.0 |
| WP 083950838.1 | 256.8 | WP 051808300.1 | 7.4 |
| KWV31814.1 | 250.0 | WP 067035690.1 | 7.0 |
| ADV92525.1 | 248.2 | WP 103564314.1 | 7.0 |
| OKK16161.1 | 243.9 | WP 080048780.1 | 6.9 |
| WP 013229249.1 | 234.6 | WP 129849678.1 | 5.7 |
| WP 131056699.1 | 231.4 | WP 125627453.1 | 5.0 |
| WP 045318561.1 | 213.8 | WP 083947829.1 | 4.8 |
| WP 047194864.1 | 208.9 | WP 083303042.1 | 4.6 |
| WP 072476921.1 | 200.9 | KOV66859.1 | 4.2 |
| WP 075737041.1 | 200.1 | SDH02982.1 | 3.9 |
| WP 065917710.1 | 198.5 | PKM30861.1 | 3.8 |
| WP 130401777.1 | 192.1 | WP 103939557.1 | 3.8 |
| WP 081971423.1 | 191.2 | WP 120781673.1 | 3.6 |
| WP 093411427.1 | 188.0 | WP 143713288.1 | 3.1 |
| WP_086849962.1 | 186.8 | WP_136529585.1 | 2.9 |
| WP 109639600.1 | 179.4 | WP 091310325.1 | 2.8 |
| WP 076469402.1 | 157.7 | GAP50618.1 | 2.5 |
| WP 086784246.1 | 130.9 | WP_020639922.1 | 2.5 |
| WP 080041749.1 | 128.4 | SCF11721.1 | 2.5 |
| WP 125313231.1 | 124.4 | KJK32958.1 | 2.4 |
| WP 120675340.1 | 118.1 | WP 130477875.1 | 2.2 |
| WP 027346283.1 | 114.2 | WP 105645204.1 | 2.1 |
| WP 155541557.1 | 113.6 | KWV30420.1 | 2.1 |
| WP 075975245.1 | 110.9 | WP 107417268.1 | 1.8 |
| WP 144126986.1 | 110.1 | EOD65379.1 | 1.7 |
| WP 078844206.1 | 109.9 | WP 092287378.1 | 1.6 |
| WP 091283529.1 | 104.6 | WP 122982115.1 | 1.5 |
| WP 103503499.1 | 104.3 | WP 107115530.1 | 1.4 |
| WP 130969140.1 | 103.0 | PWK87198.1 | 1.4 |
| WP_112669767.1 | 99.8 | KYG52038.1 | 1.3 |
| KNX38636.1 | 89.0 | ALD14029.1 | 1.3 |
| GBD22443.1 | 87.9 | OUE26840.1 | 1.3 |
| WP 083302694.1 | 87.8 | WP 132628273.1 | 1.3 |
| WP 091084084.1 | 87.5 | WP 052685689.1 | 1.2 |
| WP 132183354.1 | 78.5 | SBV25448.1 | 1.2 |
| WP 093954453.1 | 78.1 | MAM88718.1 | 1.2 |
| WP 116813002.1 | 70.2 | WP 146103553.1 | 1.1 |
| WP 089021493.1 | 68.4 | RZI82703.1 | 1.0 |
| PWK39581.1 | 64.8 | RZL00883.1 | 0.7 |
| WP 011291330.1 | 58.8 | WP 148029222.1 | 0.6 |
| WP 123685830.1 | 53.0 | WP 108710096.1 | 0.5 |
| WP 123748612.1 | 49.7 | WP 132200424.1 | 0.1 |
| | | WP 106966793.1 | 0.02 |

### Example 4. Domain Analysis of 152 Sequences of Which PET Degradation Activity was Verified

In order to explore conserved domains related to PET degradation activity, the amino acid sequences of 152 genes of which PET degradation activity was verified were aligned with the NCBI Conserved Domain Database, which has 63,113 conserved domain profiles, (Lu S, Wang J, Chitsaz F, Derbyshire MK, Geer RC, Gonzales NR, Gwadz M, Hurwitz DI, Marchler GH, Song JS, Thanki N, Yamashita RA, Yang M, Zhang D, Zheng C, Lanczycki CJ, Marchler-Bauer A. CDD/SPARCLE: the conserved domain database in 2020. Nucleic Acids Res. 2020 Jan 8;48(D1):D265-D268. doi: 10.1093/nar/gkz991. Epub 2019 Nov 28. [PubMed PMID: 31777944]) and Reverse Position-Specific BLAST program (rpsblast,]http://www.ncbi.nlm.nih.gov/Structure/cdd/wrpsb.cgi) using default options, and it was confirmed that 152 genes were aligned with the COG4188 profile with an e-value of less than 10. The multiple sequence alignment between the 152 amino acid sequences and COG4188 consensus sequence (https://ncbi.nlm.nih.gov/Structure/cdd/cdd help.shtml#CDConsensus) was performed using the default option of clustalw2 (Larkin MA, Blackshields G, Brown NP, Chenna R, McGettigan PA, McWilliam H, Valentin F, Wallace IM, Wilm A, Lopez R, Thompson JD, Gibson TJ, Higgins DG.(2007). Clustal W and Clustal X version 2.0. Bioinformatics, 23, 2947-2948.). Since the lengths of the 152 gene sequences are different, the position of the COG4188 consensus sequence was used as the representative position in the results of multiple sequence alignment. The frequencies of amino acids aligned according to the position of the COG4188 consensus sequence were calculated based on the BLOSUM62 matrix used as the default in NCBI BLAST (Mark P Styczynski; Kyle L Jensen; Isidore Rigoutsos; Gregory Stephanopoulos (2008). "BLOSUM62 miscalculations improve search performance". Nat. Biotechnol. 26 (3): 274-275. doi:10.1038/nbt0308-274. PMID 18327232. S2CID 205266180.). As a result, common mutations at 10 positions and conserved sequences at 14 positions were found, compared to the COG4188 consensus sequence. Information on the conserved sites was expressed using the PROSITE Pattern (https://prosite.expasy.org/prosuser.html) notation. In the PROSITE Pattern, '[ ]' means one or more amino acids corresponding to a specific position, for example, [ALT] means Ala or Leu or Thr. The 10 common mutation positions and 14 conserved sequence positions are as follows.
- 10 mutations information
   G62[FWY], L70P, G81[ILMV], T85P, G113[DNE], Y118[RQK], D135[QREK], Q304[HNY], L322[WFY], A330[DNE]
- 14 identical information
   G86, [LIMV]94, [AS]95, [AS]96, G98, [FWY]99, [VILM]100, [VILM]101, [DNE]157, G165, [SANT]167, G169, G170, [DNE]262

### Example 5. Analysis of Amino Acid Similarity between 152 Sequences of Which PET Degradation Activity was Verified

A similarity analysis between sequences was performed on the 152 sequences obtained through Example 1. The similarity analysis between sequences was performed using fasta36 (W.R. Pearson & D.J. Lipman PNAS (1988) 85:2444-2448) with default options. As a result of the similarity analysis between genes having PET degradation activity, when analyzing amino acid similarity using WP_039906502.1 of SEQ ID NO: 2 as a sample, 20 amino acid sequences with 80% or more similarity (minimum 82.2% IDENTITY) were identified, showing that WP_039906502.1 is the sequence with the highest level of similarity among the 152 sequences. The Accession Numbers of the sequences with 80% or more similarity are as follows: CCH19814.1, WP_110567505.1, WP_112669767.1, WP_124822775.1, WP_089021493.1, WP_139584963.1, WP_130401777.1, PIG53436.1, WP_088988822.1, WP_082377728.1, WP_091553551.1, WP_101366623.1, WP_124770494.1, KWV31814.1, WP_083302694.1, WP_091283529.1, WP_093411427.1, WP_131056699.1, WP_013736455.1, WP_130464304.1

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within the metes and bounds of the claims, or equivalents of such metes and bounds are therefore intended to be embraced by the claims.

## Claims

1. A method of identifying a polypeptide having a PET degradation activity, the method comprising the steps of:
preparing an amino acid sequence of a polypeptide (predicted polypeptide) predicted to have the PET degradation activity;
aligning the amino acid sequence of the predicted polypeptide with a reference sequence of SEQ ID NO: 1; and
determining from the alignment result whether the amino acid sequence of the predicted polypeptide has the following characteristics:
based on the reference sequence of SEQ ID NO: 1,
(1) a residue corresponding to position 62 is F, W, or Y;
(2) a residue corresponding to position 70 is P;
(3) a residue corresponding to position 81 is I, L, M, or V;
(4) a residue corresponding to position 85 is P;
(5) a residue corresponding to position 86 is G;
(6) a residue corresponding to position 94 is I, L, M, or V;
(7) a residue corresponding to position 95 is A or S;
(8) a residue corresponding to position 96 is A or S;
(9) a residue corresponding to position 98 is G;
(10) a residue corresponding to position 99 is F, W or Y;
(11) a residue corresponding to position 100 is V, L, I, or M;
(12) a residue corresponding to position 101 is V, L, I, or M;
(13) a residue corresponding to position 113 is D, N, or E;
(14) a residue corresponding to position 118 is R, Q, or K;
(15) a residue corresponding to position 135 is Q, R, E, or K;
(16) a residue corresponding to position 157 is D, N, or E;
(17) a residue corresponding to position 165 is G;
(18) a residue corresponding to position 167 is S, A, N, or T;
(19) a residue corresponding to position 169 is G;
(20) a residue corresponding to position 170 is G;
(21) a residue corresponding to position 262 is D, N, or E;
(22) a residue corresponding to position 304 is H, N, or Y;
(23) a residue corresponding to position 322 is W, F, or Y; and
(24) a residue corresponding to position 330 is D, N, or E.

2. The method of claim 1, wherein the predicted polypeptide has a plurality of amino acid sequences.

3. A polypeptide having a PET degradation activity, and having an amino acid sequence, wherein, based on the reference sequence of SEQ ID NO: 1,
(1) a residue corresponding to position 62 is F, W, or Y;
(2) a residue corresponding to position 70 is P;
(3) a residue corresponding to position 81 is I, L, M, or V;
(4) a residue corresponding to position 85 is P;
(5) a residue corresponding to position 86 is G;
(6) a residue corresponding to position 94 is I, L, M, or V;
(7) a residue corresponding to position 95 is A or S;
(8) a residue corresponding to position 96 is A or S;
(9) a residue corresponding to position 98 is G;
(10) a residue corresponding to position 99 is F, W or Y;
(11) a residue corresponding to position 100 is V, L, I, or M;
(12) a residue corresponding to position 101 is V, L, I, or M;
(13) a residue corresponding to position 113 is D, N, or E;
(14) a residue corresponding to position 118 is R, Q, or K;
(15) a residue corresponding to position 135 is Q, R, E, or K;
(16) a residue corresponding to position 157 is D, N, or E;
(17) a residue corresponding to position 165 is G;
(18) a residue corresponding to position 167 is S, A, N, or T;
(19) a residue corresponding to position 169 is G;
(20) a residue corresponding to position 170 is G;
(21) a residue corresponding to position 262 is D, N, or E;
(22) a residue corresponding to position 304 is H, N, or Y;
(23) a residue corresponding to position 322 is W, F, or Y; and
(24) a residue corresponding to position 330 is D, N, or E.

4. The polypeptide of claim 3, wherein the polypeptide having the PET degradation activity comprises motifs of the following Formulae 1 to 5:
(Formula 1) X1-X2-X3-X4-X5-X6-X7 -X8-P:
wherein X1 is F, W, or Y; and
X2 to X8 are arbitrary amino acids or absent;
(Formula 2) X1-X2-X3-X4-P-G:
wherein X1 is I, L, M, or V; and
X2 to X4 are arbitrary amino acids or absent;
(Formula 3) X1-X2-X3-X4-G-X5-X6-X7:
wherein X1 is I, L, M, or V;
X2 is A or S;
X3 is A or S;
X4 is an arbitrary amino acid or absent;
X5 is F, W, or Y;
X6 is V, L, I, or M; and
X7 is V, L, I, or M;
(Formula 4) X1-X2-X3-X4-X5-X6-X7-X8-G-X9-X10-X11-G-G
wherein X1 is D, N, or E;
X2 to X8, X9 and X11 are arbitrary amino acids or absent; and
X10 is S, A, N, or T;
(Formula 5) X1-X2-X3-X4-X5-X6-X7-X8-X9
wherein X1 is W, F, or Y; and
X2 to X8 are arbitrary amino acids or absent; and
X9 is D, N or E.

5. The polypeptide of claim 1, wherein the polypeptide has an amino acid sequence selected from WP_109362551.1, SHM40309.1, SDZ16714.1, WP_084434874.1, WP_101366623.1, WP_117215036.1, WP_132413760.1, WP_110567505.1, WP_143590710.1, WP_091553551.1, WP_124822775.1, WP_116180173.1, OGB27210.1, WP_088988822.1, WP_125191182.1, WP_085749752.1, WP_130464304.1, WP _054022242.1, WP_082377728.1, WP_132386942.1, WP_082403176.1, WP_013736455.1, WP_073887759.1, CCH19814.1, WP_141984372.1, WP_155388305.1, WP_039906502.1, WP_137976057.1, WP_132337032.1, WP_093411427.1, WP_075737041.1, WP_139584963.1, WP_088276085.1, PIG53436.1, WP_045318561.1, WP_078759821.1,AEV21261.1, WP_047194864.1, WP_083950838.1, KWV31814.1, ADV92525.1, EOD65379.1, WP_013229249.1, WP_131056699.1, WP_072476921.1, WP_065917710.1, WP_130401777.1, WP_081971423.1, WP_086849962.1, WP_109639600.1, WP_150303381.1, WP_076469402.1, WP_086784246.1, WP_125313231.1, WP_120675340.1, WP_027346283.1, WP_155541557.1, WP_075975245.1, WP_144126986.1, WP_078844206.1, WP_091283529.1, WP_103503499.1, WP_130969140.1, WP_112669767.1, KNX38636.1, GBD22443.1, WP_083302694.1, WP_091084084.1, WP_093954453.1, WP_116813002.1, WP_089021493.1, PWK39581.1, WP_011291330.1, WP_123685830.1, WP_123748612.1, WP_084400763.1, WP_132183354.1, ADV92527.1, WP_014689902.1, WP_093935592.1, RZS34008.1, WP_138709315.1, WP_141569747.1, 1JFR_A, WP_124770494.1, 4WFI_A, WP_143737449.1, WP_080041749.1, CBY05529.1, WP_111700265.1, OKK16161.1, WP_143320421.1, WP_084578793.1, WP_104613137.1, WP_104115406.1, SPF04022.1, WP_119701604.1, WP_120326365.1, PWW60275.1, WP_086668955.1, WP_092528534.1, WP_063729606.1, 3VIS_A, WP_138698985.1, WP_145923095.1, WP_068752972.1, MBA55398.1, WP_123888242.1, WP_083724990.1, WP_044499735.1, WP_051808300.1, WP_067035690.1, WP_103564314.1, WP_129849678.1, WP_125627453.1, WP_083947829.1, WP_083303042.1, KOV66859.1, SDH02982.1, PKM30861.1, WP_103939557.1, WP_120781673.1, WP_136529585.1, WP_091310325.1, GAP50618.1, SCF11721.1, WP_130477875.1, KWV30420.1, WP_143713288.1, WP_107417268.1, WP_106966793.1, WP_092287378.1, WP_080048780.1, WP_122982115.1, PWK87198.1, KYG52038.1, OUE26840.1, WP_052685689.1, SBV25448.1, MAM88718.1, RZI82703.1, WP_107115530.1, KJK32958.1, WP_146103553.1, WP_132628273.1, ALD14029.1, RZL00883.1, WP_148029222.1, WP_105645204.1, WP_108710096.1, WP_020639922.1 and WP_132200424.1.

6. A composition comprising the polypeptide of any one of claims 3 to 5.

7. The composition of claim 6, wherein the composition is for degrading PET.

8. A polynucleotide encoding the polypeptide of any one of claims 3 to 5.

9. A host cell comprising the polypeptide of any one of claims 3 to 5; a polynucleotide encoding the polypeptide; a nucleic acid construct comprising the polynucleotide; and/or a vector comprising the polynucleotide or the nucleic acid construct.

10. A method of preparing a polypeptide having a PET degradation activity, the method comprising the steps of:
culturing the host cell of claim 9; and
recovering the polypeptide which is expressed in the culturing step.

11. A method of degrading a polyester, the method comprising treating the polyester with any one or more of the polypeptide of any one of claims 3 to 5; a host cell expressing the polypeptide; and a composition comprising the polypeptide.

12. The method of claim 11, wherein the polyester is PET.

13. A method of producing mono(2-hydroxymethyl) terephthalate (MHET), terephthalic acid (TPA), and/or ethylene glycol (EG), the method comprising bringing a polyester into contact with any one or more of the polypeptide of any one of claims 3 to 5; a host cell expressing the polypeptide; and a composition comprising the polypeptide.

14. The method of claim 13, wherein the polyester is PET.

15. The method of claim 13, further comprising the step of recovering the produced MHET, TPA, and/or EG.

16. A method of producing a polyester, the method comprising the step of synthesizing the polyester using MHET, TPA, and/or EG produced by the method of any one of claims 13 to 15.

17. The method of claim 16, wherein the polyester is PET.

18. Use of the polypeptide of any one of claims 3 to 5, a host cell expressing the polypeptide, or a composition comprising the polypeptide in degrading PET.

19. Use of the polypeptide of any one of claims 3 to 5 or a composition comprising the polypeptide for the reaction with a polyester in producing mono(2-hydroxymethyl) terephthalate (MHET), terephthalic acid (TPA), and/or ethylene glycol (EG).
